(19) **Europäisches Patentamt / European Patent Office / Office européen des brevets**

(11) **EP 4 670 707 A1**

(12) **EUROPEAN PATENT APPLICATION**
published in accordance with Art. 153(4) EPC

(43) Date of publication:
**31.12.2025  Bulletin 2026/01**

(21) Application number: **24760386.3**

(22) Date of filing: **21.02.2024**

(51) International Patent Classification (IPC):
*A61K 8/29* [(2006.01)]   *A61K 8/04* [(2006.01)]
*A61K 8/31* [(2006.01)]   *A61K 8/37* [(2006.01)]
*A61K 8/73* [(2006.01)]   *A61Q 17/04* [(2006.01)]

(52) Cooperative Patent Classification (CPC):
A61K 8/04; A61K 8/29; A61K 8/31; A61K 8/37;
A61K 8/73; A61Q 17/04

(86) International application number:
**PCT/JP2024/006147**

(87) International publication number:
**WO 2024/177088 (29.08.2024 Gazette 2024/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.02.2023  JP 2023025334**

(71) Applicant: **Kao Corporation**
**Chuo-ku**
**Tokyo 103-8210 (JP)**

(72) Inventors:
- **HORIKOSHI Mina**
  **Tokyo 131-8501 (JP)**
- **ISHITA Mio**
  **Tokyo 131-8501 (JP)**

(74) Representative: **Hoffmann Eitle**
**Patent- und Rechtsanwälte PartmbB**
**Arabellastraße 30**
**81925 München (DE)**

(54) **OIL-IN-WATER EMULSION COSMETIC**

(57)    Provided is an oil-in-water emulsified cosmetic including the following components (A), (B), and (C): (A) a fatty acid-coated titanium oxide having a surface tension of 40 mN/m or less; (B) a volatile hydrocarbon oil having a viscosity at 37.8°C of 7 mPa·s or less (B1) and an ester oil (B2); and (C) an oil gelling agent, a content of the component (A) being 3 mass% or more and 20 mass% or less, a content of the component (B) being 5 mass% or more and 43 mass% or less, and a mass ratio of the content of the component (B) to a content of an oil-phase component [component (B)/oil-phase component] being 0.50 or more.

Fig. 1

**Description**

Field of the Invention

The present invention relates to an oil-in-water emulsified cosmetic.

Background of the Invention

In recent years, as the importance of protection from UV rays in daily life increases, the demand for sunscreen cosmetics is also expanding. In response to such market demands, oil-in-water emulsified sunscreen cosmetics which have refreshing use impression and are easy to use continuously are being developed.
For these oil-in-water emulsified cosmetics, UV absorbers and UV scattering agents are used in order to enhance their UV protection effects. Particularly as UV scattering agents, metal oxide powders, such as titanium oxide and zinc oxide, are used.
For example, for the purpose of providing a skin care cosmetic which can effectively protect the skin from various external irritants including UV rays as well as air pollutants and acidic liquids, i.e., an anti-pollution cosmetic, WO 2014/136993 (Patent Literature 1) describes a skin care cosmetic containing from 0.001 to 10 mass% of magnesium aluminometasilicate and from 1 to 40 mass% of a UV protection agent, in which a metal oxide powder, such as titanium oxide or zinc oxide, is used as the UV protection agent.

Summary of the Invention

The present invention relates to an oil-in-water emulsified cosmetic comprising the following components (A), (B), and (C):

(A) a fatty acid-coated titanium oxide having a surface tension of 40 mN/m or less;
(B) a volatile hydrocarbon oil having a viscosity at 37.8°C of 7 mPa·s or less (B1) and an ester oil (B2); and
(C) an oil gelling agent,

a content of the component (A) being 3 mass% or more and 20 mass% or less,
a content of the component (B) being 5 mass% or more and 43 mass% or less, and
a mass ratio of the content of the component (B) to a content of an oil-phase component [component (B)/oil-phase component] being 0.50 or more.

Brief Description of Drawings

Fig. 1 schematically shows a method for evaluating the effect of inhibiting the adhesion of air toxic substances.
Fig. 2 shows electron micrographs of the cross-sections of the coating films of Example 1 and Comparative Example 2.

Detailed Description of the Invention

It found that the technique of Patent Literature 1 cannot provide sufficient UV protection effects, and when wearing a mask as a measure against hay fever and infectious diseases, the durability of the UV protection effect is not sufficient due to the friction between the mask and the cosmetic coating film formed on the skin surface. It is required by alleviating discomfort caused by the mask fibers coming into contact with the skin surface.
Furthermore, in recent years, various health problems to the human body is caused by pollen from cedar, cypress, and the like, air pollutants, such as smoke and powder dust, and toxic substances suspended in the air, such as yellow sand (hereinafter also referred to as "air toxic substances"). Among such air toxic substances, particulate matter with a diameter of 2.5 μm or less, known as PM2.5, is composed of carbon components, sulfates, nitrates, ammonium salts, and the like, and PM2.5 and yellow sand are known to cause circulatory and respiratory system diseases by inhalation. It pointed out that PM2.5, yellow sand, and pollen adhere to or penetrate the skin, thereby causing skin problems. For these reasons, there is a strong demand for cosmetics which can effectively prevent the adhesion of air toxic substances to the skin.
It found that when one attempts to enhance the UV protection effect and the effect of inhibiting the adhesion of air toxic substances, good use impression may not be acquired when applying the cosmetic.

The present invention relates to an oil-in-water emulsified cosmetic which has good use impression during application, has an excellent UV protection effect and an excellent effect of inhibiting the adhesion of air toxic substances, and can form a cosmetic coating film which has durability of the UV protection effect. This oil-in-water emulsified cosmetic can also reduce discomfort caused by wearing a mask.

The present inventors found that when a fatty acid-coated titanium oxide whose surface tension is equal to or less than a predetermined value, a volatile hydrocarbon oil whose viscosity is equal to or less than a predetermined value and an ester oil as oil agents, and an oil gelling agent are contained, and when the contents of the fatty acid-coated titanium oxide and oil agents are within predetermined ranges, and the mass ratio of the content of the oil agents to the content of an oil-phase component is equal to or greater than a predetermined value, it is possible to provide an oil-in-water emulsified cosmetic which has good use impression during application, has an excellent UV protection effect and an excellent effect of inhibiting the adhesion of air toxic substances, and can form a cosmetic coating film which has durability of the UV protection effect.

The present invention relates to an oil-in-water emulsified cosmetic which has good use impression during application, has an excellent UV protection effect and an excellent effect of inhibiting the adhesion of air toxic substances, and can form a cosmetic coating film which has durability of the UV protection effect. This oil-in-water emulsified cosmetic can also reduce discomfort caused by wearing a mask.

[Oil-in-water emulsified cosmetic]

The oil-in-water emulsified cosmetic of the present invention (hereinafter also referred to as "the emulsified cosmetic of the present invention" or "the emulsified cosmetic") contains the following components (A), (B), and (C):

(A) a fatty acid-coated titanium oxide having a surface tension of 40 mN/m or less;
(B) a volatile hydrocarbon oil having a viscosity at 37.8°C of 7 mPa·s or less (B1) and an ester oil (B2); and
(C) an oil gelling agent,

a content of the component (A) being 3 mass% or more and 20 mass% or less,
a content of the component (B) being 5 mass% or more and 43 mass% or less, and
a mass ratio of the content of the component (B) to a content of an oil-phase component [component (B)/oil-phase component] being 0.50 or more.

In the present invention, the phrase "containing component X" or "containing component X" also means "being blended with component X."

In the present invention, the term "volatile" means that the amount of evaporation at 25°C for 6 hours is more than 20%, as measured by the following method.

Measurement method: Filter paper with a diameter of 90 mm is placed in a glass Petri dish with a diameter of 120 mm, 1 g of a sample is placed on the filter paper, and the sample is then stored in a room at 65% RH (25°C) for 6 hours. The mass of the sample before and after storage is measured, and the amount of evaporation is calculated by the following formula:

Amount of evaporation (%) = (mass of sample before storage - mass of sample after storage)/mass of sample before storage $\times$ 100

In the present invention, the "oil-phase component" refers to an oil agent and an oil-soluble component, and also includes components which are dissolved and blended with the oil agent and oil-soluble component during preparation of the oil-in-water emulsified cosmetic (provided that powder components are excluded). The oil-phase component does not include a surfactant. The "oil-soluble component" as mentioned herein refers to a component which has a non-uniform appearance when visually observed in a mixture obtained by mixing 100 parts by mass of water and 3 parts by mass of the component at 25°C. The phrase that "the mixture has a non-uniform appearance" means that separation is visually observed. That is, the oil-phase component in the present invention contains the components (B) and (C), as well as optionally a component (D), optionally a component (G), and optionally a component (H). When the oil-in-water emulsified cosmetic of the present invention dissolves components other than the components (B), (C), (D), (G), and (H) in the oil phase during preparation, those components are also included.

In the present invention, the "air toxic substances" refers to pollen from cedar, cypress, and the like; smoke and powder dust, such as sulfur oxides, dust, and nitrogen oxides, automobile exhaust gas, toxic air pollutants, such as benzene, trichloroethylene, and tetrachloroethylene, and air pollutants, such as particles containing volatile organic compounds (VOCs); and toxic substances suspended in the air, such as yellow sand (including PM2.5).

The emulsified cosmetic of the present invention has good use impression during application, has an excellent UV protection effect and an excellent effect of inhibiting the adhesion of air toxic substances, and can form a cosmetic coating film which has durability of the UV protection effect. Although the reason for this is unclear, it is considered as follows.

In the present invention, the surface tension of the fatty acid-coated titanium oxide is equal to or less than a predetermined value; thus, the aggregation of the fatty acid-coated titanium oxide can be suppressed, and the fatty acid-coated titanium oxide can be uniformly applied to the target object, such as skin. In addition, it is considered that since the fatty acid-coated titanium oxide is contained in the emulsified cosmetic at a predetermined content, a UV protection effect can be effectively exhibited, and the durability of the UV protection effect can be improved. Furthermore, the surface tension of the fatty acid-coated titanium oxide is equal to or less than a predetermined value, and the fatty acid-coated titanium oxide has low wettability with the volatile hydrocarbon oil, whose viscosity is equal to or less than a predetermined value, and the ester oil, which are contained as oil agents; thus, irregularities derived from the fatty acid-coated titanium oxide can be formed on the surface of the target object, such as the skin. As a result, it is considered that when air toxic substances come into contact with the fatty acid-coated titanium oxide applied to the surface of the target object, the contact area can be reduced, and the adhesion of the air toxic substances can be effectively inhibited.

Since the present invention contains an oil gelling agent, the viscosity of the emulsified cosmetic can be adjusted to enhance uniform spreadability. In addition, the content of the fatty acid-coated titanium oxide and the content of the volatile hydrocarbon oil, whose viscosity is equal to or less than a predetermined value, and the ester oil as oil agents are each within a predetermined range, and the mass ratio of the content of the oil agents to the content of an oil-phase component is equal to or greater than a predetermined value. Probably for these reasons, when the emulsified cosmetic of the present invention is applied, the emulsified cosmetic is prevented from sinking into depressions in the skin, such as pores and skin grooves. Therefore, the thickness of the cosmetic coating film formed on the skin increases, thereby providing good use impression during application, more effectively exhibiting a UV protection effect and an effect of inhibiting the adhesion of air toxic substances, and further improving the durability of the UV protection effect. Furthermore, it is considered that when wearing a mask, irritation to the skin caused by the mask fibers can be further reduced, thereby reducing discomfort when wearing the mask.

<Component (A): Fatty acid-coated titanium oxide having a surface tension of 40 mN/m or less>

From the viewpoint of improving the UV protection effect and the durability of the UV protection effect, from the viewpoint of improving the effect of inhibiting the adhesion of air toxic substances, and from the viewpoint of reducing the discomfort when wearing a mask, the emulsified cosmetic of the present invention contains, as a component (A), a fatty acid-coated titanium oxide having a surface tension of 40 mN/m or less (hereinafter also referred to as "the component (A) ") .

The surface tension of the component (A) is 40 mN/m or less, preferably 39 mN/m or less, more preferably 38 mN/m or less, further more preferably 37 mN/m or less, and even more preferably 36 mN/m or less, from the viewpoint of improving the UV protection effect and the durability of the UV protection effect, and is preferably 20 mN/m or more, more preferably 25 mN/m or more, further more preferably 30 mN/m or more, and even more preferably 33 mN/m or more, from the same viewpoint as above. More specifically, the surface tension of the component (A) is preferably from 20 to 40 mN/m, more preferably from 25 to 39 mN/m, further more preferably from 30 to 38 mN/m, even more preferably from 30 to 37 mN/m, even more preferably from 30 to 36 mN/m, and even more preferably from 33 to 36 mN/m, from the same viewpoint as above.

The surface tension of the component (A) is measured by the method described in the Examples.

The fatty acid-coated titanium oxide as the component (A) is preferably one which is subjected to fatty acid treatment using fatty acid as a surface treatment agent.

The surface treatment agent may be used singly or in combination of two or more.

Preferred examples of the surface treatment agent used for fatty acid treatment include linear or branched higher fatty acids having 12 or more and 22 or less carbon atoms. Among these, from the viewpoint of adjusting surface tension and improving the UV protection effect and the durability of the UV protection effect, the surface treatment agent used for fatty acid treatment is more preferably a linear or branched higher fatty acid having 14 or more and 22 or less carbon atoms, further more preferably a linear or branched higher fatty acid having 16 or more and 20 or less carbon atoms, and even more preferably one or more members selected from the group consisting of stearic acid and isostearic acid.

The fatty acid-coated titanium oxide as the component (A) is preferably treated with, as a surface treatment agent, a hydrous oxide of a metal, such as silica, hydrous silica, or aluminum, in combination with the fatty acid mentioned above, from the viewpoint of reducing photocatalytic activity. From the viewpoint of reducing photocatalytic activity, adjusting surface tension, and improving the UV protection effect and the durability of the UV protection effect, the fatty acid-coated titanium oxide as the component (A) is preferably aluminum hydroxide/stearic acid-coated titanium oxide microparticles.

From the viewpoint of reducing the photocatalytic activity, adjusting the surface tension, and improving the UV protection effect and the durability of the UV protection effect, the fatty acid coating amount in the component (A) is preferably 0.1 mass% or more, more preferably 1 mass% or more, further more preferably 3 mass% or more, and even more preferably 5 mass% or more, and is preferably 40 mass% or less, more preferably 30 mass% or less, and further more preferably 25 mass% or less.

The content of titanium oxide ($TiO_2$) in the component (A) is preferably 60 mass% or more, and more preferably 65 mass% or more, and is preferably 95 mass% or less, more preferably 90 mass% or less, and further more preferably 85 mass% or less.

In the present invention, the surface tension, mass, and average primary particle size, described later, of the component (A) refer to the surface tension, the mass, and the average primary particle size thereof including the surface treatment agent.

The shape of the component (A) may be, for example, spherical, flaky, plate-like, rod-like, spindle-like, needle-like, or irregular, but any shape can be used.

The average primary particle size of the component (A) is preferably 3 nm or more, more preferably 5 nm or more, and further more preferably 10 nm or more, and is preferably 100 nm or less, more preferably 80 nm or less, further more preferably 50 nm or less, even more preferably 30 nm or less, even more preferably 25 nm or less, and even more preferably 20 nm or less, from the viewpoints of adjusting surface tension and improving the UV protection effect and durability of the UV protection effect, and from the viewpoint of versatility. More specifically, the average primary particle size of the component (A) is preferably from 3 to 100 nm, more preferably from 5 to 100 nm, further more preferably from 5 to 80 nm, even more preferably from 5 to 50 nm, even more preferably from 5 to 50 nm, even more preferably from 5 to 30 nm or less, even more preferably from 5 to 25 nm, even more preferably from 5 to 20 nm, and even more preferably from 10 to 20 nm, from the same viewpoints as above.

The average primary particle size of the component (A) in the present invention can be determined from images observed with a transmission electron microscope (TEM). Specifically, the average primary particle size can be determined by observing the particle size at a magnification of 50 000 times using a TEM, measuring the maximum minor axes of 300 primary particles in the observed image, and calculating the number average value thereof. When the component (A) has a shape other than a flaky or plate-like shape, the maximum minor axis as mentioned herein refers to, among the minor axes perpendicular to the major axes, a minor axis having the maximum length. When the component (A) has a flaky or plate-like shape, the average primary particle size is determined by measuring the thicknesses of 300 primary particles in an image observed under the same conditions as above, and calculating the number average value thereof. Specifically, the average primary particle size is measured by the method described in the Examples.

The component (A) can be prepared by treating titanium oxide treated with a hydrous oxide of a metal, such as silica, hydrous silica, or aluminum, as a component (Ax) using the fatty acid mentioned above by a known method.

The preferred embodiment and measurement method of the average primary particle size as the component (Ax) used in the surface treatment mentioned above are the same as the preferred embodiment and measurement method for the component (A).

Commercial products of the fatty acid-coated titanium oxide used as the component (A) include MPT series (e.g., "MPT-171" and "MPT-186") (manufactured by Ishihara Sangyo Kaisha, Ltd.), STR series (e.g., "STR-100C-LF") (manufactured by Sakai Chemical Industry Co., Ltd.), and MT series (e.g., "MT-N1" and "MT-01") (manufactured by Tayca Corporation).

The content of the component (A) in the emulsified cosmetic of the present invention is 3 mass% or more, preferably 5 mass% or more, more preferably 8 mass% or more, further more preferably 10 mass% or more, and even more preferably 12 mass% or more, from the viewpoint of improving the UV protection effect and the durability of the UV protection effect, from the viewpoint of improving the effect of inhibiting the adhesion of air toxic substances, and from the viewpoint of reducing discomfort when wearing a mask, and is 20 mass% or less, preferably 18 mass% or less, more preferably 16 mass% or less, and further more preferably 14 mass% or less, from the viewpoint of improving the use impression during application, and from the viewpoint of improving emulsion stability. More specifically, from the viewpoint of improving the UV protection effect and the durability of the UV protection effect, from the viewpoint of improving the effect of inhibiting the adhesion of air toxic substances, from the viewpoint of reducing discomfort when wearing a mask, and from the viewpoint of improving emulsion stability, the content of the component (A) in the emulsified cosmetic of the present invention is from 3 to 20 mass%, preferably from 3 to 18 mass%, more preferably from 5 to 18 mass%, further more preferably from 8 to 18 mass%, even more preferably from 10 to 18 mass%, even more preferably from 12 to 18 mass%, even more preferably from 12 to 16 mass%, and even more preferably from 12 to 14 mass%.

The mass ratio of the content of the component (A) to the content of an oil-phase component in the emulsified cosmetic of the present invention [component (A)/oil-phase component] is preferably 0.20 or more, more preferably 0.25 or more, further more preferably 0.30 or more, even more preferably 0.35 or more, and even more preferably 0.40 or more, from the viewpoint of improving the UV protection effect and the durability of the UV protection effect, from the viewpoint of improving the effect of inhibiting the adhesion of air toxic substances, and from the viewpoint of reducing discomfort when wearing a mask, and is preferably 0.80 or less, more preferably 0.70 or less, further more preferably 0.60 or less, and even more preferably 0.50 or less, from the viewpoint of improving the use impression during application, and from the viewpoint of improving emulsion stability. More specifically, the mass ratio [component (A)/oil-phase component] is preferably from 0.20 to 0.80, more preferably from 0.25 to 0.70, further more preferably from 0.30 to 0.60, even more preferably from 0.35 to 0.50, and even more preferably from 0.40 to 0.50, from the same viewpoints as above.

<Component (B): Volatile hydrocarbon oil having a viscosity at 37.8°C of 7 mPa·s or less (B1) and ester oil (B2) >

The emulsified cosmetic of the present invention contains, as a component (B), a volatile hydrocarbon oil having a viscosity at 37.8°C of 7 mPa·s or less (B1) (hereinafter also referred to as "the component (B1)") and an ester oil (B2) (hereinafter also referred to as "the component (B2)") (hereinafter also referred to as "the component (B)"), from the viewpoint of improving the effect of inhibiting the adhesion of air toxic substances, and from the viewpoint of reducing discomfort when wearing a mask.
The components (B1) and the components (B2) each can be used singly or in combination of two or more.

[Component (B1): Volatile hydrocarbon oil having a viscosity at 37.8°C of 7 mPa·s or less]

The component (B1) is a volatile hydrocarbon oil having a viscosity at 37.8°C of 7 mPa·s or less, makes the component (A) disperse, and does not wet the powder surface, thus contributing to the development of the effect of inhibiting the adhesion of air toxic substances. Blending the component (B1) in addition to the component (B2) can reduce the discomfort when using a mask and improve the use impression.
For example, the component (B1) is preferably one or more members selected from the group consisting of paraffin-based volatile hydrocarbon oils, such as n-decane, n-undecane, and n-dodecane; isoparaffin-based volatile hydrocarbon oils, such as isodecane, isododecane, isohexadecane, and hydrogenated polyisobutene (light liquid isoparaffin); and cyclic paraffin hydrocarbon oils, such as cyclodecane and cyclododecane. Among these, from the viewpoint of improving the effect of inhibiting the adhesion of air toxic substances, and from the viewpoint of reducing discomfort when wearing a mask, the component (B1) is more preferably light liquid isoparaffin.
The viscosity at 37.8°C of the component (B1) is 7 mPa·s or less, preferably 6 mPa·s or less, more preferably 5 mPa·s or less, and further more preferably 4 mPa·s or less, and is preferably 0.3 mPa·s or more, more preferably 0.5 mPa·s or more, and further more preferably 1 mPa·s or more. More specifically, the viscosity at 37.8°C of the component (B1) is preferably from 0.3 to 7 mPa·s, more preferably from 0.5 to 6 mPa·s, further more preferably from 1 to 5 mPa·s, and even more preferably from 1 to 4 mPa·s.
The viscosity at 37.8°C of the component (B1) is measured with a B-type viscometer using rotor No. 1 at a rotational speed of 60 rpm and a temperature of 37.8°C, as described in the Examples.
Commercial products of the light liquid isoparaffin include Parleam 4 (manufactured by NOF Corporation).

[Component (B2): Ester oil]

The component (B2) is an ester oil, disperses the component (A), and can contribute to the development of the effect of inhibiting the adhesion of air toxic substances. Examples of the ester oil include synthetic ester oils, natural fats/oils, and the like, such as an ester of monovalent carboxylic acid and monohydric alcohol, an ester of monovalent carboxylic acid and polyhydric alcohol, and an ester of polyvalent carboxylic acid and monohydric alcohol.
The ester of monovalent carboxylic acid and monohydric alcohol is, for example, an ester of the following formula (1):

$$R^1\text{-COO-}R^2 \qquad (1)$$

In the formula (1), $R^1$ represents a linear or branched alkyl or alkenyl group having 1 or more and 25 or less carbon atoms in which some of the hydrogen atoms are optionally substituted with hydroxy groups, or an aromatic group-containing hydrocarbon group having 6 or more and 24 or less carbon atoms, and $R^2$ represents a linear or branched alkyl or alkenyl group having 1 or more and 30 or less carbon atoms.
When $R^1$ is an alkyl group or an alkenyl group, the number of carbon atoms in $R^1$ is preferably 7 or more, and is preferably 23 or less, more preferably 21 or less, further more preferably 19 or less, and even more preferably 17 or less.
When $R^1$ is an aromatic group-containing hydrocarbon group, the number of carbon atoms in $R^1$ is preferably 6 or more, and is preferably 22 or less, and more preferably 20 or less.
The number of carbon atoms in $R^2$ is preferably 2 or more, and is preferably 28 or less, more preferably 24 or less, further more preferably 22 or less, and even more preferably 20 or less.
Specific examples of the ester of the formula (1) include one or more members selected from the group consisting of cetyl isooctanoate (cetyl 2-ethylhexanoate), stearyl isooctanoate, isononyl isononanoate, isotridecyl isononanoate, hexyl laurate, isostearyl laurate, butyl myristate, isopropyl myristate, decyl myristate, isotridecyl myristate, isocetyl myristate, isostearyl myristate, 2-octyldodecyl myristate, isopropyl palmitate, 2-ethylhexyl palmitate, isostearyl palmitate, 2-hexyldecyl palmitate, 2-ethylhexyl stearate, 2-hexyldecyl stearate, isopropyl isostearate, 2-hexyldecyl isostearate, ethyl oleate, isodecyl oleate, oleyl oleate, 2-octyldodecyl oleate, ethyl linoleate, isopropyl linoleate, lanolin acetate, castor oil fatty acid methyl (methyl ricinoleate), and alkyl benzoate (number of carbon atoms in alkyl: from 12 to 15).

The ester of monovalent carboxylic acid and monohydric alcohol is also, for example, an ester of the following formula (2):

$$R^3\text{-COO-}(AO)_n\text{-}R^4 \qquad (2)$$

In the formula (2), $R^3$ represents a linear or branched alkyl or alkenyl group having 1 or more and 25 or less carbon atoms in which some of the hydrogen atoms are optionally substituted with hydroxy groups, and $R^4$ represents an aromatic group-containing hydrocarbon group having 6 or more and 24 or less carbon atoms. AO represents an alkyleneoxy group having 2 or more and 4 or less carbon atoms, and n represents 1 or more and 50 or less as the average number of moles added.

$R^3$ is an alkyl group having preferably 7 or more carbon atoms, and having preferably 23 or less, more preferably 21 or less, and further more preferably 19 or less carbon atoms.

$R^4$ is an aromatic group-containing hydrocarbon group preferably having 6 or more carbon atoms, and having preferably 22 or less, more preferably 20 or less, and further more preferably 18 or less carbon atoms, and is even more preferably a benzyl group.

The alkyleneoxy group of AO is preferably a propyleneoxy group, and n is preferably 1 or more and 10 or less, and more preferably 1 or more and 5 or less.

Specific examples of the ester of the formula (2) include an ester of myristic acid and a 3-mole propylene oxide adduct of benzyl alcohol (Crodamol STS, manufactured by Croda Inc.) and an ester of 2-ethylhexanoic acid and a 3-mole propylene oxide adduct of benzyl alcohol (Crodamol SFX, manufactured by Croda Inc.).

The ester of monovalent carboxylic acid and polyhydric alcohol is, for example, an ester of the following formula (3):

$$R^5\text{-}(OCOR^6)_p \qquad (3)$$

In the formula (3), $R^5$ represents a polyhydric alcohol residue, and preferably a hydrocarbon group having 2 or more and 10 or less carbon atoms, $R^6$ represents a monovalent carboxylic acid residue having 1 or more and 25 or less carbon atoms, and p represents an integer of 2 or more and 10 or less.

$R^5$ may have an ether bond, but is preferably a linear or branched hydrocarbon group having 2 or more and 10 or less carbon atoms. p is preferably the same number as the number of hydroxy groups in the polyhydric alcohol mentioned above.

$R^6$ is an alkyl group having preferably 5 or more, and more preferably 7 or more carbon atoms, and from the same viewpoint as above, having preferably 23 or less, more preferably 21 or less, further more preferably 19 or less, and even more preferably 17 or less carbon atoms.

Examples of the ester of the formula (3) include propylene glycol dicaprate, propylene glycol dioleate, neopentyl glycol dicaprate, neopentyl glycol di-2-ethylhexanoate, di(caprylic/capric)propanediol, propanediol diisostearate, ethylene glycol di-2-ethylhexanoate, caprylic/capric triglyceride, glyceryl tri(2-ethylhexanoate) (triethylhexanoin), glyceryl tri-2-heptylundecanoate, trimethylolpropane tri-2-ethylhexylate, trimethylolpropane triisostearate, pentaerythritol tetra-2-ethylhexanoate, and natural fats/oils.

Examples of natural fats/oils include triglycerides, such as avocado oil, olive oil, sesame oil, rice bran oil, safflower oil, soybean oil, corn oil, rapeseed oil, castor oil, cottonseed oil, and mink oil.

The ester of polyvalent carboxylic acid and monohydric alcohol is also, for example, an ester of the following formula (4):

$$R^7\text{-}(COOR^8)_q \qquad (4)$$

In the formula (4), $R^7$ represents a polyvalent carboxylic acid residue having 2 or more and 10 or less carbon atoms, and $R^8$ represents a monohydric alcohol residue having 1 or more and 25 or less carbon atoms, and q is an integer of 2 or more and 10 or less. q is preferably the same number as the number of carboxy groups in the polyvalent carboxylic acid mentioned above.

The number of carbon atoms in $R^8$ is preferably 3 or more, and is preferably 23 or less, more preferably 21 or less, further more preferably 19 or less, from the same viewpoint as above.

Specific examples include diisostearyl malate, di-2-ethylhexyl succinate, diisobutyl adipate, di-2-heptylundecyl adipate, di-2-ethylhexyl sebacate, diisopropyl sebacate, tri-2-ethylhexyl trimellitate, and the like.

Among these, from the viewpoint of improving the effect of inhibiting the adhesion of air toxic substances, and from the viewpoint of reducing discomfort when wearing a mask, the component (B2) is preferably the ester of the formula (1) or (3); more preferably one or more members selected from the group consisting of a monoester of linear or branched fatty acid having 8 or more and 18 or less carbon atoms and linear or branched monohydric alcohol having 2 or more and 24 or less carbon atoms, a triester of branched fatty acid having 6 or more and 18 or less carbon atoms and glycerin, a tetraester of branched fatty acid having 6 or more and 18 or less carbon atoms and pentaerythritol, and a diester of linear or branched fatty acid having 6 or more and 18 or less carbon atoms and branched dihydric alcohol having 2 or more and 10 or less carbon atoms; and more preferably one or more members selected from the group consisting of neopentyl glycol di-2-ethylhexanoate, triethylhexanoin, isotridecyl isononanoate, isononyl isononanoate, and neopentyl glycol dicaprate.

The content of the component (B) in the emulsified cosmetic of the present invention is 5 mass% or more, preferably 10 mass% or more, more preferably 15 mass% or more, and further more preferably 20 mass% or more, from the viewpoint of improving the effect of inhibiting the adhesion of air toxic substances, and from the viewpoint of reducing discomfort when wearing a mask, and is 43 mass% or less, preferably 35 mass% or less, more preferably 30 mass% or less, and further more preferably 25 mass% or less, from the viewpoint of improving the effect of inhibiting the adhesion of air toxic substances, from the viewpoint of reducing discomfort when wearing a mask, and from the viewpoint of improving the use impression during application. More specifically, the content of the component (B) in the emulsified cosmetic of the present invention is from 5 to 43 mass%, preferably from 10 to 35 mass%, more preferably from 15 to 35 mass%, further more preferably from 15 to 30 mass%, even more preferably from 20 to 30 mass%, and even more preferably from 20 to 25 mass%, from the same viewpoints as above.

The mass ratio of the content of the component (A) to the content of the component (B) in the emulsified cosmetic of the present invention [component (A)/component (B)] is preferably 0.20 or more, more preferably 0.30 or more, and further more preferably 0.40 or more, and is preferably 1.0 or less, more preferably 0.90 or less, further more preferably 0.80 or less, even more preferably 0.70 or less, and even more preferably 0.60 or less, from the viewpoint of improving the UV protection effect and the durability of the UV protection effect, from the viewpoint of improving the effect of inhibiting the adhesion of air toxic substances, and from the viewpoint of reducing discomfort when wearing a mask. More specifically, the mass ratio [component (A)/component (B)] is preferably from 0.20 to 1.0, more preferably from 0.20 to 0.90, further more preferably from 0.30 to 0.90, even more preferably from 0.40 to 0.80, even more preferably from 0.40 to 0.70, and even more preferably from 0.40 to 0.60, from the same viewpoints as above.

The mass ratio of the content of the component (B) to the content of the oil-phase component in the emulsified cosmetic of the present invention [component (B)/oil-phase component] is 0.50 or more, preferably 0.60 or more, more preferably 0.70 or more, further more preferably 0.80 or more, and even more preferably 0.85 or more, and is preferably 0.98 or less, more preferably 0.95 or less, and further more preferably 0.92 or less, from the viewpoint of improving the UV protection effect and the durability of the UV protection effect, from the viewpoint of improving the effect of inhibiting the adhesion of air toxic substances, and from the viewpoint of reducing discomfort when wearing a mask. More specifically, the mass ratio [component (B)/oil-phase component] is preferably from 0.50 to 0.98, more preferably from 0.60 to 0.98, further more preferably from 0.70 to 0.95, even more preferably from 0.80 to 0.95, even more preferably from 0.85 to 0.95, and even more preferably from 0.85 to 0.92, from the same viewpoints as above.

The component (B) is a combination of a volatile hydrocarbon oil (B1) and an ester oil (B2), from the viewpoint of improving the UV protection effect and the durability of the UV protection effect, from the viewpoint of improving the effect of inhibiting the adhesion of air toxic substances, and from the viewpoint of reducing discomfort when wearing a mask. The mass ratio of the content of the component (B1) to the content of the component (B2) in the emulsified cosmetic of the present invention [component (B1)/component (B2)] is preferably 0.15 or more, more preferably 0.25 or more, and further more preferably 0.35 or more, from the viewpoint of improving the UV protection effect and the durability of the UV protection effect, from the viewpoint of improving the effect of inhibiting the adhesion of air toxic substances, and from the viewpoint of reducing discomfort when wearing a mask, and is preferably 3.0 or less, more preferably 2.0 or less, further more preferably 1.7 or less, even more preferably 1.0 or less, even more preferably 0.70 or less, and even more preferably 0.50 or less, from the same viewpoints as above. More specifically, the mass ratio [component (B1)/component (B2)] is preferably from 0.15 to 3.0, more preferably from 0.25 to 2.0, further more preferably from 0.35 to 1.7, even more preferably from 0.35 to 1.0, even more preferably from 0.35 to 0.70, and even more preferably from 0.35 to 0.50, from the same viewpoints as above.

The mass ratio of the content of the component (B1) to the content of the component (A) in the emulsified cosmetic of the present invention [component (B1)/component (A)] is preferably 0.30 or more, more preferably 0.40 or more, and further more preferably 0.50 or more, and is preferably 2.5 or less, more preferably 2.0 or less, further more preferably 1.5 or less, and even more preferably 1.0 or less, from the viewpoint of improving the UV protection effect and the durability of the UV protection effect, from the viewpoint of improving the effect of inhibiting the adhesion of air toxic substances, from the viewpoint of reducing discomfort when wearing a mask, and from the viewpoint of improving the use impression during application. More specifically, the mass ratio [component (B1)/component (A)] is preferably from 0.30 to 2.5, more preferably from 0.30 to 2.0, further more preferably from 0.30 to 1.5, even more preferably from 0.30 to 1.0, even more preferably 0.40 or more and 1.0 or less, and even more preferably 0.50 or more and 1.0 or less, from the same viewpoints as above.

The mass ratio of the content of the component (B2) to the content of the component (A) in the emulsified cosmetic of the present invention [component (B2)/component (A)] is preferably 0.30 or more, more preferably 0.50 or more, further more preferably 0.80 or more, and even more preferably 1.0 or more, and is preferably 1.5 or less, from the viewpoint of improving the UV protection effect and the durability of the UV protection effect, from the viewpoint of improving the effect of inhibiting the adhesion of air toxic substances, from the viewpoint of reducing discomfort when wearing a mask, and from the viewpoint of improving the use impression during application. More specifically, the mass ratio [component (B2)/component (A)] is preferably from 0.30 to 1.5, more preferably from 0.50 to 1.5, further more preferably from 0.80 to 1.5, and even more preferably from 1.0 to 1.5, from the same viewpoints as above.

<Component (C): Oil gelling agent>

The emulsified cosmetic of the present invention contains, as a component (C), an oil gelling agent (hereinafter also referred to as "the component (C)"), from the viewpoint of facilitating viscosity adjustment, from the viewpoint of enhancing uniform spreadability and increasing the thickness of the cosmetic coating film formed on the skin, from the viewpoint of providing good use impression during application, more effectively exhibiting a UV protection effect and an effect of inhibiting the adhesion of air toxic substances, and further improving the durability of the UV protection effect, and from the viewpoint of reducing discomfort when wearing a mask.

The components (C) can be used singly or in combination of two or more.

For example, the component (C) is preferably one or more members selected from the group consisting of an organically modified clay mineral, a dextrin fatty acid ester, a metal soap, a sucrose fatty acid ester, and an amino acid-based gelling agent.

The organically modified clay mineral is not particularly limited as long as it is used for general cosmetics. Examples thereof include cation-modified clay minerals obtained by treating layered clay minerals, such as bentonite, laponite, hectorite, montmorillonite, and magnesium aluminum silicate, with a quaternary ammonium salt cationic surfactant. Examples of such cation-modified clay minerals include dimethyl distearyl ammonium hectorite, dimethyl distearyl ammonium bentonite, benzyl dimethyl stearyl ammonium hectorite, and the like.

Commercial products of the organically modified clay mineral include "Bentone 38," "Bentone 38VCG," and "Bentone 27," manufactured by Elementis Japan KK.

The organically modified clay mineral can also be used as premix gel diluted with a solvent, from the viewpoint of improving the workability and from the viewpoint of improving the thickening effect. Specifically preferred is premix gel in which the organically modified clay mineral is dispersed in a solvent in advance. The solvent is not limited as long as it can be gelled with the organically modified clay mineral. From the viewpoint of improving the thickening effect, octyldodecanol, mineral oils, and the like are preferred. From the viewpoint of efficiently dispersing the organically modified clay mineral, it is preferable to further contain polar additives, such as propylene carbonate, ethanol, water, and various surfactants.

The content of the organically modified clay mineral in the premix gel is preferably 5 mass% or more, more preferably 8 mass% or more, and further more preferably 10 mass% or more, and is preferably 25 mass% or less, more preferably 20 mass% or less, and further more preferably 18 mass% or less, from the viewpoint of improving workability, from the viewpoint of improving the thickening effect, and from the viewpoint of preventing oil separation from the premix gel itself.

Commercial products of the premix gel include "Bentone Gel EUGV," "Bentone Gel MIOV," "Bentone Gel VS-5 PCV," "Bentone Gel PTM V," and "Bentone Gel GTCC V," manufactured by Elementis Japan KK.

The dextrin fatty acid ester is not limited as long as it is used for general cosmetics, and is preferably an ester of fatty acid having 8 or more and 24 or less carbon atoms and dextrin, and more preferably an ester of fatty acid having 14 or more and 20 or less carbon atoms and dextrin, from the viewpoint of facilitating viscosity adjustment, and from the viewpoint of enhancing spreadability and the UV protection effect. An ester of dextrin with an average degree of polymerization of 3 or more and 150 or less is also preferred.

Specific examples of the dextrin fatty acid ester include dextrin palmitate, dextrin stearate, dextrin palmitate/stearate, dextrin oleate, dextrin isopalmitate, dextrin isostearate, dextrin myristate, dextrin palmitate/2-ethylhexanoate, and the like.

Among these, from the viewpoint of facilitating viscosity adjustment, and from the viewpoint of enhancing spreadability and the UV protection effect, preferred is one or more members selected from the group consisting of dextrin palmitate, dextrin myristate, and dextrin palmitate/2-ethylhexanoate; and more preferred is one at least containing dextrin palmitate.

Commercial products of the dextrin fatty acid ester include dextrin palmitates ("Rheopearl KL2," "Rheopearl KS2," and "Rheopearl TL2"), dextrin palmitate/2-ethylhexanoate "Rheopearl TT2," and dextrin myristate "Rheopearl MKL2," manufactured by Chiba Flour Milling Co., Ltd.

The amino acid-based gelling agent is not particularly limited as long as it is used for general cosmetics. Specifically, dibutyl lauroyl glutamide and dibutyl ethylhexanoyl glutamide are preferred.

Commercial products of the amino acid-based gelling agent include dibutyl lauroyl glutamide "GP-1" and dibutyl ethylhexanoyl glutamide "EB-21," manufactured by Ajinomoto Co., Inc.

The amino acid-based gelling agent can also be used as premix gel diluted and dissolved in a solvent, from the viewpoint of improving workability, and from the viewpoint of improving the thickening effect. Specifically preferred is premix gel in which the amino acid-based gelling agent is dissolved in a solvent in advance. The solvent is not limited as long as it can be gelled with the amino acid-based gelling agent. Octyldodecanol, isostearic acid, and the like are preferred, from the viewpoint of improving the thickening effect.

The content of the amino acid-based gelling agent in the premix gel is preferably 10 mass% or more, more preferably 15 mass% or more, and further more preferably 20 mass% or more, and is preferably 45 mass% or less, more preferably 40 mass% or less, and further more preferably 36 mass% or less, from the viewpoint of improving workability, from the viewpoint of improving the thickening effect, and from the viewpoint of preventing oil separation from the premix gel itself.

Commercial products of the premix gel include "AJK-OD2046" (containing 20 mass% of an amino acid-based gelling agent) and "AJK-IS3613" (containing 36 mass% of an amino acid-based gelling agent), manufactured by Kokyu Alcohol Kogyo Co., Ltd.

The content of the component (C) in the emulsified cosmetic of the present invention is preferably 0.1 mass% or more, more preferably 0.3 mass% or more, further more preferably 0.5 mass% or more, and even more preferably 0.8 mass% or more, from the viewpoint of facilitating viscosity adjustment, from the viewpoint of enhancing uniform spreadability, providing good use impression during application, more effectively exhibiting a UV protection effect and an effect of inhibiting the adhesion of air toxic substances, and further improving the durability of the UV protection effect, and from the viewpoint of reducing discomfort when wearing a mask, and is preferably 3 mass% or less, more preferably 2 mass% or less, further more preferably 1.5 mass% or less, and even more preferably 1.2 mass% or less, from the viewpoint of providing good use impression during application and more effectively exhibiting an effect of inhibiting the adhesion of air toxic substances, and from the viewpoint of reducing discomfort when wearing a mask. More specifically, the content of the component (C) in the emulsified cosmetic of the present invention is preferably from 0.1 to 3 mass%, more preferably from 0.3 to 2 mass%, further more preferably from 0.5 to 1.5 mass%, and even more preferably from 0.8 to 1.2 mass%.

<Component (D): Film-forming agent>

The emulsified cosmetic of the present invention may contain, as a component (D), a film-forming agent (hereinafter also referred to as "the component (D)"), within a range which does not impair the effects of the present invention.

The component (D) is not limited as long as it is used for general cosmetics, and is, for example, one or more members selected from the group consisting of silicone-based resins, such as trimethylsiloxysilicate, partially crosslinked organopolysiloxane, trimethylsiloxysilylpropylcarbamic acid, fluorine-modified silicone, alkyl-modified silicone, (meth) acrylic-modified silicone, and a silicone dendrimer-modified resin compound; rosin acid-based resins, such as pentaerythritol rosinate and glyceryl rosinate; candelilla resins (e.g., a candelilla wax fraction having a resin component content of 65 mass% or more obtained by fractional extraction of resin components contained in candelilla wax); polyvinyl isobutyl ether; polyisobutylene; and solid waxes (provided that alkyl-modified silicone is excluded).

The "solid waxes" as mentioned herein refer to waxes which exhibit solid properties in an environment of 25°C under 1 atmosphere. Examples of such waxes include waxes having a melting point of 61°C or higher.

In the present specification, the melting point is measured by either Method 1, Method 2, or Method 3 of the General Test Methods in the Japanese Standards of Quasi-Drug Ingredients. Which method to use should be in accordance with the measurement method specified in the Japanese Standards of Quasi-Drug Ingredients for each ingredient, if any. If no measurement method is specified, the measurement method is selected taking into account the melting point. Specifically, it is common to use Method 1 when the melting point is high enough to greatly exceed 80°C, Method 2 for solid fats with a lower melting point, and Method 3 for those called paste oils in catalogs and the like; however, any method which allows for measurement can be used.

Examples of waxes having a melting point of 61°C or higher include mineral-based hydrocarbon waxes, such as ozokerite and ceresin; petroleum-based hydrocarbon waxes, such as paraffin and microcrystalline wax; hydrocarbon-based waxes, such as Fischer-Tropsch wax, polyethylene wax, $\alpha$-olefins having 28 or more carbon atoms, and other synthetic hydrocarbon waxes; plant waxes, such as carnauba wax, candelilla wax, rice wax, and sunflower wax; animal waxes, such as beeswax and whale wax; synthetic beeswax, and the like.

The components (D) can be used singly or in combination of two or more.

The content of the component (D) in the emulsified cosmetic of the present invention is preferably 0 mass% or more, and is preferably 1 mass% or less, more preferably 0.7 mass% or less, further more preferably 0.4 mass% or less, even more preferably 0.2 mass% or less, and even more preferably 0 mass%, from the viewpoint of improving the use impression during application. More specifically, the content of the component (D) in the emulsified cosmetic of the present invention is preferably from 0 to 1 mass%, more preferably from 0 to 0.7 mass%, further more preferably from 0 to 0.4 mass%, even more preferably from 0 to 0.2 mass%, and even more preferably 0 mass%, from the viewpoint of improving the use impression during application.

In the present invention, even if the content of the component (D) is within the above range, the thickness of the cosmetic coating film formed on the skin can be increased, and when wearing a mask, irritation to the skin caused by the mask fibers coming into contact with the skin surface can be reduced; thus, it is considered that discomfort when wearing the mask can also be reduced. When the content of the component (D) is within the above range, the sticky or heavy feeling after application can be suppressed without inhibiting the effects of the present invention, and it is easy to wash off when washing the face.

<Component (E): Surfactant>

The emulsified cosmetic of the present invention preferably further contains, as a component (E), a surfactant (hereinafter also referred to as "the component (E)"), from the viewpoint of obtaining good emulsion stability.

The component (E) is not particularly limited, and any of nonionic, anionic, cationic, and amphoteric surfactants can be used, but nonionic surfactants are preferred. The components (E) can be used singly or in combination of two or more. Among these, the component (E) is more preferably one or more members selected from the group consisting of ceramide, polyhydroxy fatty acid, an ester of pentaerythritol and polyhydroxy fatty acid, a sorbitan fatty acid ester, a polyoxyalkylene sorbitan fatty acid ester, a polyoxyalkylene fatty acid ester, a polyoxyalkylene alkyl ether, a polyoxyalkylene alkenyl ether, an alkyl glucoside, an alkyl glyceryl ether, a sucrose fatty acid ester, a glycerin fatty acid ester, a polyglycerin fatty acid ester, a polyoxyalkylene hydrogenated castor oil, an alkyl saccharide, an alkylamine oxide, an alkyl amidoamine oxide, and polyether-modified silicone.

The ceramide can be one or more members selected from the group consisting of natural ceramides and pseudo-ceramides. Specifically, the ceramides described in JP-A-2020-063239 can be used.

The alkyl groups in the polyoxyalkylene alkyl ether, alkyl glucoside, alkyl glyceryl ether, alkyl saccharide, alkylamine oxide, and alkyl amidoamine oxide, the alkenyl group in the polyoxyalkylene alkenyl ether, and the fatty acids in the polyhydroxy fatty acid, ester of pentaerythritol and polyhydroxy fatty acid, sorbitan fatty acid ester, polyoxyalkylene sorbitan fatty acid ester, polyoxyalkylene fatty acid ester, sucrose fatty acid ester, glycerin fatty acid ester, and polyglycerin fatty acid ester preferably have 8 or more and 22 or less carbon atoms, more preferably 10 or more and 20 or less carbon atoms, and further more preferably 12 or more and 20 or less carbon atoms.

The emulsified cosmetic of the present invention preferably contains, as the component (E), a surfactant in which the calculated value of the common logarithm (hereinafter referred to as "clogP") of the partition coefficient P (1-octanol/water) between 1-octanol and water is 10 or more (hereinafter also referred to as "the component (E1)"), from the viewpoint of preventing the thickening of the emulsified cosmetic and improving the use impression when applying the emulsified cosmetic.

The clogP of the component (E1) is "LogP (cLogP)" calculated by the method described in A. Leo, Comprehensive Medicinal Chemistry, Vol. 4 C. Hansch, P.G. Sammens, J.B. Taylor, and C.A. Ramsden, Eds., p. 295, Pergamon Press, 1990, and is a value calculated by the program CLOGP v4.01.

When the emulsified cosmetic of the present invention contains aluminum hydroxide/stearic acid-coated titanium oxide microparticles as the component (A) and a copolymer containing a structural unit derived from acryloyldimethyltauric acid as a component (F) described later, and when the emulsified cosmetic also contains the component (E1), it is considered to be able to prevent the orientation of the component (A) present in the oil phase to the oil/water interface, to prevent the thickening of the emulsified cosmetic due to the interaction between a water-soluble polymer present in the aqueous phase and the component (A), and to improve the use impression when applying the emulsified cosmetic.

Specific examples of the component (E1) include N-(hexadecyloxyhydroxypropyl)-N-hydroxyethylhexadecanamide (clogP: 13.4), Polyglyceryl-6 Octacaprylate ("Salacos HG-8," manufactured by Nisshin OilliO Group, Ltd.), diisostearyl malate ("Cosmol 222," manufactured by Nisshin OilliO Group, Ltd.), polyglyceryl diisostearate ("Cosmol 42V" (clogP: 15.7), manufactured by Nisshin OilliO Group, Ltd.), polyglyceryl triisostearate ("Cosmol 43N," manufactured by Nisshin OilliO Group, Ltd.), ditrimethylolpropane (isostearate/sebacate) oligoester ("Salacos DT-318S," manufactured by Nisshin OilliO Group, Ltd.), sorbitan distearate ("Sunsoft No. 63C-C" (clogP: 17.6), manufactured by Taiyo Kagaku Co., Ltd.), dipentaerythrityl tripolyhydroxystearate ("Salacos WO-6" (clogP: 35.7), manufactured by Nisshin OilliO Group, Ltd.), and polyhydroxystearic acid ("Salacos HS-6C" (clogP: 34.2), manufactured by Nisshin OilliO Group, Ltd.).

Among these, the component (E1) is preferably one or more members selected from the group consisting of N-(hexadecyloxyhydroxypropyl)-N-hydroxyethylhexadecanamide (clogP: 13.4), polyglyceryl diisostearate (clogP: 15.7), sorbitan distearate (clogP: 17.6), dipentaerythrityl tripolyhydroxystearate (clogP: 35.7), and polyhydroxystearic acid (clogP: 34.2); more preferably one or more members selected from the group consisting of dipentaerythrityl tripolyhydroxystearate (clogP: 35.7) and polyhydroxystearic acid (clogP: 34.2); and further more preferably dipentaerythrityl tripolyhydroxystearate (clogP: 35.7).

The content of the component (E) in the emulsified cosmetic of the present invention is preferably 0.5 mass% or more, more preferably 1 mass% or more, and further more preferably 2 mass% or more, and is preferably 6 mass% or less, more preferably 5 mass% or less, and further more preferably 4 mass% or less, from the viewpoint of improving emulsion stability. More specifically, the content of the component (E) in the emulsified cosmetic of the present invention is preferably from 0.5 to 6 mass%, more preferably from 1 to 5 mass%, and further more preferably from 2 to 4 mass%, from the same viewpoint as above.

When the emulsified cosmetic of the present invention contains the component (E1) as the component (E), the content of the component (E1) in the emulsified cosmetic of the present invention is preferably 0.5 mass% or more, more preferably 1 mass% or more, and further more preferably 2 mass% or more, and is preferably 6 mass% or less, more preferably 5 mass% or less, further more preferably 4 mass% or less, from the viewpoint of preventing the thickening of the emulsified cosmetic and improving the use impression during application. More specifically, the content of the component (E1) in the emulsified cosmetic of the present invention is preferably from 0.5 to 6 mass%, more preferably from 1 to 5 mass%, and further more preferably from 2 to 4 mass%, from the same viewpoint as above.

When the emulsified cosmetic of the present invention contains dipentaerythrityl tripolyhydroxystearate (clogP: 35.7) as the component (E1), the mass ratio of the content of dipentaerythrityl tripolyhydroxystearate to the content of the component (A) in the emulsified cosmetic of the present invention [dipentaerythrityl tripolyhydroxystearate/component (A)] is preferably 0.01 or more, more preferably 0.02 or more, and further more preferably 0.04 or more, and is preferably 0.15 or less, more preferably 0.10 or less, further more preferably 0.08 or less, and even more preferably 0.07 or less, from the viewpoint of improving the UV protection effect and the durability of the UV protection effect, preventing the thickening of the emulsified cosmetic, and improving the use impression during application. More specifically, the mass ratio [dipentaerythrityl tripolyhydroxystearate/component (A)] is preferably from 0.01 to 0.15, more preferably from 0.01 to 0.10, further more preferably from 0.02 to 0.08, and even more preferably from 0.04 to 0.07, from the same viewpoint as above.

<Component (F): Water-soluble polymer>

The emulsified cosmetic of the present invention preferably further contains, as a component (F), a water-soluble polymer (hereinafter also referred to as "the component (F)"), from the viewpoint of emulsion stability.

The component (F) is not limited as long as it is used for general cosmetics. Any of natural, semi-synthetic, and synthetic polymers can be used.

Examples of natural polymers include xanthan gum, carrageenan, alginic acid, and the like. Among these, from the viewpoint of moisturizing properties, xanthan gum is preferred.

Examples of semi-synthetic polymers include modified polysaccharides, such as hydroxycellulose, hydroxyethylcellulose, hydroxypropylcellulose, sodium carboxymethylcellulose, methylcellulose, hydroxymethylcellulose, and cationized cellulose.

Examples of synthetic polymers include one or more acrylic polymers selected from the group consisting of a homopolymer of (meth)acrylic acid, (meth)acrylate, or a (meth)acrylamide-based monomer, and a copolymer containing one or more structural units derived from these monomers; polyvinylpyrrolidone, polyvinyl alcohol, cationized polyvinylpyrrolidone, and the like.

Examples of acrylic polymers include copolymers containing one or more acrylamide-based monomer-derived structural units selected from the group consisting of crosslinked polyacrylic acid (carbomer), polyacrylic acid, alkyl acrylate/methacrylate copolymers and salts thereof (e.g., alkyl-modified carboxyvinyl polymers), polyacrylamide, acrylamide, and acryloyldimethyltauric acid.

Among these, from the viewpoint of moisturizing properties, and from the viewpoint of emulsion stability, the component (F) is preferably one or more members selected from the group consisting of xanthan gum and an acrylic polymer, and preferably xanthan gum from the viewpoint of moisturizing properties, or an acrylic polymer from the viewpoint of emulsion stability. The acrylic polymer is one or more members selected from the group consisting of (1) crosslinked polyacrylic acid (carbomer), (2) an alkyl-modified carboxyvinyl polymer, (3) polyacrylamide, and (4) a copolymer containing one or more acrylamide-based monomer-derived structural units selected from the group consisting of acrylamide and acryloyldimethyltauric acid.

Examples of the crosslinked polyacrylic acid (carbomer) (1) include "Carbopol 910," "Carbopol 934," "Carbopol 940," "Carbopol 941," "Carbopol 980," and "Carbopol 981" (all manufactured by Lubrizol Advanced Materials); and "Synthalen K" and "Synthalen L" (manufactured by 3V SIGMA).

Examples of the alkyl-modified carboxyvinyl polymer (2) include copolymers of acrylic acid and alkyl methacrylate (preferably with an alkyl group having 8 or more and 30 or less carbon atoms), such as "PEMULEN TR-1 POLYMER," "PEMULEN TR-2 POLYMER," "Carbopol ETD 2020," and "Carbopol 1382" (all manufactured by Lubrizol Advanced Materials).

Examples of the polyacrylamide (3) include "SEPIGEL 305" (polyacrylamide, (C13-14) Isoparaffin, Laureth-7, water) and "SEPIGEL 501" ((C13-14) Isoparaffin, mineral oil, polyacrylamide, Polysorbate 85, sodium polyacrylate).

Preferred examples of the copolymer containing one or more acrylamide-based monomer-derived structural units selected from the group consisting of acrylamide and acryloyldimethyltauric acid (hereinafter also referred to as "AMPS") (4) include one or more members selected from the group consisting of an (acrylic acid/acrylamide) copolymer, an (acrylic acid/AMPS) copolymer, a (dimethylacrylamide/AMPS) copolymer, an (acrylic acid/AMPS/dimethylacrylamide) copolymer, a (hydroxyethyl acrylate/AMPS) copolymer, an (AMPS/vinylpyrrolidone) copolymer, an (AMPS/vinylformamide) copolymer, an (AMPS/polyoxyethylene alkyl ester of methacrylic acid (average number of moles of ethylene oxide added: from 10 to 30)) copolymer, and salts thereof.

More specific examples of the copolymer containing one or more acrylamide-based monomer-derived structural units selected from the group consisting of acrylamide and acryloyldimethyltauric acid (AMPS) (4) include a (hydroxyethyl acrylate/sodium acryloyldimethyl taurate) copolymer (INCI name: Hydroxyethyl Acrylate/Sodium Acryloyldimethyl Taurate Copolymer), a (sodium acrylate/sodium acryloyldimethyl taurate) copolymer (INCI name: Sodium Acrylate/Sodium Acryloyldimethyl Taurate Copolymer), a (sodium acrylate/acryloyldimethyltaurate/dimethylacrylamide) crosspolymer (INCI name: Sodium Acrylate/Acryloyldimethyltaurate/Dimethylacrylamide Crosspolymer), an (ammonium acryloyldimethyltaurate/vinylpyrrolidone) copolymer (INCI name: Ammonium Acryloyldimethyltaurate/VP Copolymer), an acrylic acid/acrylic acid amide/sodium acrylate/sodium acryloyldimethyl taurate copolymer (INCI name: Polyacrylate-13), an (ammonium acryloyldimethyltaurate/dimethylacrylamide/lauryl methacrylate/laureth-4 methacrylate) crosspolymer (INCI name: Polyacrylate Crosspolymer-6), an (ammonium acryloyldimethyltaurate/beheneth-25 methacrylate) crosspolymer (INCI name: Ammonium Acryloyldimethyltaurate/Beheneth-25 Methacrylate Crosspolymer), and an (ammonium acryloyldimethyltaurate/steareth-25 methacrylate) crosspolymer (INCI name: Ammonium Acryloyldimethyltaurate/Steareth-25 Methacrylate Crosspolymer).

Examples of commercial products of the copolymer containing one or more acrylamide-based monomer-derived structural units selected from the group consisting of acrylamide and acryloyldimethyltauric acid (AMPS) (4) include "SIMULGEL EG QD" ((sodium acrylate/sodium acryloyldimethyl taurate) copolymer, isohexadecane, Polysorbate 80, water, sorbitan oleate), "SEPIMAX ZEN" (Polyacrylate Crosspolymer-6, t-butanol), "SEPINOV EMT 10" ((hydroxyethyl acrylate/sodium acryloyldimethyl taurate) copolymer, sorbitan isostearate, Polysorbate 60), "SIMULGEL NS" ((hydroxyethyl acrylate/sodium acryloyldimethyl taurate) copolymer, squalane, Polysorbate 60, water, sorbitan isostearate), "SIMULGEL FL" (water, (hydroxyethyl acrylate/sodium acryloyldimethyl taurate) copolymer, isohexadecane, Polysorbate 60, sorbitan isostearate), "SEPIPLUS S" ((hydroxyethyl acrylate/sodium acryloyldimethyl taurate) copolymer, polyisobutene, PEG-7 trimethylolpropane coconut oil alkyl ether, water, sorbitan isostearate), and "SEPIPLUS 400" (Polyacrylate-13, polyisobutene, water, Polysorbate 20, sorbitan isostearate) (all manufactured by SEPPIC); "Aristoflex AVC" ((ammonium acryloyldimethyltaurate/VP) copolymer, t-butanol, water), "Aristoflex HMB" ((ammonium acryloyldimethyltaurate/beheneth-25 methacrylate) crosspolymer, t-butanol, water), and "Aristoflex HMS" ((ammonium acryloyldimethyltaurate/steareth-25 methacrylate) crosspolymer) (all manufactured by Clariant).

The content of the component (F) in the emulsified cosmetic of the present invention is preferably 0.05 mass% or more, more preferably 0.10 mass% or more, further more preferably 0.30 mass% or more, and even more preferably 0.50 mass% or more, and is preferably 3.0 mass% or less, more preferably 2.0 mass% or less, and further more preferably 1.0 mass% or less, from the viewpoint of emulsion stability. More specifically, the content of the component (F) in the emulsified cosmetic of the present invention is preferably from 0.05 to 3.0 mass%, more preferably from 0.10 to 2.0 mass%, further more preferably from 0.30 to 1.0 mass%, and even more preferably from 0.50 to 1.0 mass%, from the same viewpoint as above.

(Aqueous medium)

The emulsified cosmetic of the present invention preferably contains, as an aqueous medium, one or more members selected from the group consisting of water, monohydric alcohols, and polyhydric alcohols.

Usable examples of water include deionized water, distilled water, highly pure water, and ultrapure purified water.

Examples of monohydric alcohols include saturated monohydric alcohols having 2 or more and 4 or less carbon atoms, such as ethanol, propanol, isopropanol, and t-butyl alcohol.

Examples of polyhydric alcohols include divalent alcohols, such as ethylene glycol, diethylene glycol, triethylene glycol, polyethylene glycol (an average molecular weight of less than 650), 1,2-propanediol (propylene glycol), 1,3-propanediol (propanediol), dipropylene glycol, polypropylene glycol (an average molecular weight of less than 650), 1,3-butanediol (1,3-butylene glycol), and polybutylene glycol (an average molecular weight of less than 650); and trivalent alcohols, such as glycerin, diglycerin, polyglycerin.

The aqueous media can be used singly or in combination of two or more.

The content of the aqueous medium in the emulsified cosmetic of the present invention may be within a range in which the emulsified cosmetic can be an oil-in-water cosmetic, and the content is generally 98.7 mass% or less. The content of the aqueous medium in the emulsified cosmetic of the present invention may be the balance of all active ingredients in the emulsified cosmetic.

In addition to the components (A) to (F), the emulsified cosmetic of the present invention may appropriately contain optional components, which are used depending on the purpose of cosmetics, within a range which does not impair the effects of the present invention. Such optional components include oil agents, water-soluble polymers, UV absorbers, neutralizing agents, pH adjusters, disinfectants, anti-inflammatory agents, preservatives, colorants, chelating agents, skin whitening agents, antiperspirants, insect repellents, bioactive components, salts, antioxidants, and fragrances, other than the components (A) to (F).

<Component (G): Oil agent other than component (B)>

The emulsified cosmetic of the present invention may contain an oil agent other than the component (B) (hereinafter also referred to as "the component (G)"), within a range which does not impair the effects of the present invention, from the viewpoint of improving emulsion stability. The components (G) can be used singly or in combination of two or more.

The component (G) is, for example, a liquid oil agent.

In the present specification, the term "liquid" refers to a state which has fluidity in an environment of 25°C under 1 atmosphere, that is, a state where the temperature is equal to or higher than the melting point (for an amorphous substance which does not have a melting point, a state where the temperature is equal to or higher than the softening point). The term "solid" refers to a state which does not have fluidity in an environment of 25°C under 1 atmosphere, that is, a state where the temperature is below the melting point (for an amorphous substance which does not have a melting point, a state where the temperature is below the softening point).

For example, such a liquid oil agent is preferably one or more members selected from the group consisting of a silicone oil, a non-volatile hydrocarbon oil, a higher alcohol, and a higher fatty acid.

The silicone oil is preferably at least one member selected from the group consisting of dimethyl silicone and modified silicone, more preferably dimethyl silicone, further more preferably dimethylpolysiloxane having a kinematic viscosity at 25°C of 1 $mm^2$/s or more and 20 $mm^2$/s or less, and even more preferably dimethylpolysiloxane having a kinematic viscosity at 25°C of 1 $mm^2$/s or more and 20 $mm^2$/s or less.

The kinematic viscosity at 25°C of the silicone oil can be measured in accordance with ASTM D 445-46T or JIS Z 8803 using an Ubbelohde viscometer.

Commercial products of the silicone oil include "KF-96A-1CS" (octamethyltrisiloxane), "KF-96A-6cs" (dimethylpolysiloxane), "KF-96A-10cs" (dimethylpolysiloxane), and "KF-96A-20cs" (dimethylpolysiloxane), manufactured by Shin-Etsu Chemical Co., Ltd.

Preferred examples of the non-volatile hydrocarbon oil include hydrocarbon oils having a viscosity at 37.8°C of more than 7 mPa·s. Among these, the hydrocarbon oil is preferably liquid isoparaffin, from the viewpoint of improving the effect of inhibiting the adhesion of air toxic substances, and from the viewpoint of reducing discomfort when wearing a mask.

Commercial products of the liquid isoparaffin include "Parleam EX" (liquid isoparaffin) manufactured by NOF Corporation.

Examples of the higher alcohol include branched or unsaturated higher alcohols having 12 or more and 24 or less carbon atoms, such as 2-octyldodecanol, isostearyl alcohol, and oleyl alcohol. Preferred among these are branched or unsaturated higher monohydric alcohols having 12 or more and 24 or less carbon atoms.

Examples of the higher fatty acid include liquid fatty acids having 12 or more and 22 or less carbon atoms, such as oleic acid and isostearic acid.

The emulsified cosmetic of the present invention may also contain, as the component (G), an oil agent which is solid in an environment of 25°C under 1 atmosphere, from the viewpoint of emulsion stability.

Examples of solid oil agents include solid hydrocarbon oils, such as vaseline; solid higher alcohols, such as cetyl alcohol, stearyl alcohol, and behenyl alcohol; and solid higher fatty acids having 12 or more and 22 or less carbon atoms, such as lauric acid, myristic acid, palmitic acid, stearic acid, and behenic acid.

The content of the component (G) in the emulsified cosmetic of the present invention is preferably 0 mass% or more, and is preferably 10 mass% or less, more preferably 7 mass% or less, further more preferably 5 mass% or less, even more preferably 4 mass% or less, and even more preferably 3 mass% or less, and may be 0 mass%, from the viewpoint of improving emulsion stability, within a range which does not impair the effects of the present invention. More specifically, the content of the component (G) in the emulsified cosmetic of the present invention is preferably from 0 to 10 mass%, more preferably from 0 to 7 mass%, further more preferably from 0 to 5 mass%, even more preferably from 0 to 4 mass%, and even more preferably from 0 to 3 mass%, from the same viewpoints as above.

<Component (H): UV absorber>

From the viewpoint of further improving the UV protection effect, the emulsified cosmetic of the present invention may contain one or more members selected from the group consisting of liquid organic UV absorbers and solid organic UV absorbers (hereinafter also referred to as "the component (H)").

Examples of liquid organic UV absorbers include cinnamate-based UV absorbers, such as 2-ethylhexyl p-methoxycinnamate, 2-ethoxyethyl p-methoxycinnamate, isopropyl p-methoxycinnamate and diisopropyl cinnamate ester mixture, and methylbis (trimethylsiloxy)silylisopentyl trimethoxycinnamate; p-aminobenzoate-based UV absorbers, such as amyl p-dimethylaminobenzoate and 2-ethylhexyl p-dimethylaminobenzoate; salicylate-based UV absorbers, such as ethylene glycol salicylate, 2-ethylhexyl salicylate, butyloctyl salicylate, benzyl salicylate, and homomenthyl salicylate; octocrylene; dimethicodiethyl benzalmalonate; a copolymer of adipic acid and neopentyl glycol end-capped with octyldodecanol or cyanodiphenylpropenoic acid, and the like. These liquid organic UV absorbers can be contained singly or in combination of two or more.

Examples of commercially available liquid organic UV absorbers include "UVINUL MC80" (2-ethylhexyl p-methoxycinnamate) (manufactured by BASF); "PARSOL 340" (octocrylene) (manufactured by DSM Nutrition Japan K.K.); "PARSOL EHS" (2-ethylhexyl salicylate), "PARSOL HMS" (homomenthyl salicylate), and "PARSOL SLX" (Polysilicone-15 (INCI Name: Polysilicone-15) (dimethicodiethyl benzalmalonate)) (all manufactured by DSM); and "Polycrylene" (Polyester-8 (INCI Name: Polyester-8) (a copolymer of adipic acid and neopentyl glycol end-capped with octyldodecanol or cyanodiphenylpropenoic acid)) (manufactured by Hallstar).

Examples of solid organic UV absorbers include 4-tert-butyl-4'-methoxydibenzoylmethane, hexyl 2-(4-diethylamino-2-hydroxybenzoyl)benzoate, 2,4,6-tris[4-(2-ethylhexyloxycarbonyl)anilino]-1,3,5-triazine, 2-hydroxy-4-methoxybenzophenone, drometrizole trisiloxane, 2,4-bis{[4-(2-ethylhexyloxy)-2-hydroxy]phenyl}-6-(4-methoxyphenyl)-1,3,5-triazine, 2-ethylhexyl dimethoxybenzylidene dioxoimidazolidine propionate, and the like. These water-insoluble organic UV absorbers can be contained singly or in combination of two or more.

Examples of commercially available solid organic UV absorbers include "PARSOL 1789" (4-tert-butyl-4'-methoxydibenzoylmethane) (manufactured by DSM); "UVINUL A PLUS" (hexyl 2-(4-diethylamino-2-hydroxybenzoyl) benzoate), "UVINUL T-150" (2,4,6-tris[4-(2-ethylhexyloxycarbonyl)anilino]-1,3,5-triazine), "UVINUL M40" (2-hydroxy-4-methoxybenzophenone), and "TINOSORB S" (2,4-bis{[4-(2-ethylhexyloxy)-2-hydroxy]phenyl}-6-(4-methoxyphenyl)-1,3,5-triazine) (all manufactured by BASF); "Mosacare A440" (drometrizole trisiloxane) (manufactured by UFC Corporation); "Soft Shade DH" (2-ethylhexyl dimethoxybenzylidene dioxoimidazolidine propionate) (manufactured by Ajinomoto Co., Inc.), and the like.

The content of the component (H) in the emulsified cosmetic of the present invention may be preferably 3 mass% or less, more preferably 2 mass% or less, further more preferably 1 mass% or less, even more preferably 0.5 mass% or less, even more preferably 0.3 mass% or less, and even more preferably 0 mass%. More specifically, the content of the component (H) in the emulsified cosmetic of the present invention is preferably from 0 to 3 mass%, more preferably from 0 to 2 mass%, further more preferably from 0 to 1 mass%, even more preferably from 0 to 0.5 mass%, and even more preferably from 0 to 0.3 mass%.

The emulsified cosmetic of the present invention can be suitably used as skin cosmetics, such as sunscreen cosmetics, facial cleansers, cleansing cosmetics, packs, and massage cosmetics; and hair cosmetics, such as shampoos, rinses, and conditioners. Among these, the emulsified cosmetic of the present invention has an excellent UV protection effect, and is thus preferably applied to sunscreen cosmetics (e.g., lotions, creams, emulsions, and serums), suntans, cosmetic bases, foundations, and the like for sunscreen use.

The emulsified cosmetic of the present invention can be in the form of an emulsion, cream, gel, paste, solid, multi-layer, or the like, and can also be used as a sheet, spray, or mousse.

The emulsified cosmetic of the present invention contains the component (A) uniformly emulsified and dispersed therein, has an excellent UV protection effect and an excellent effect of inhibiting the adhesion of air toxic substances to the skin, has durability of the UV protection effect when wearing a mask, and can form a cosmetic coating film which can reduce discomfort caused by wearing a mask. Therefore, the emulsified cosmetic of the present invention is particularly useful as a sunscreen cosmetic.

(Method for producing oil-in-water emulsified cosmetic)

The method for producing the emulsified cosmetic of the present invention is not particularly limited, and any known method can be appropriately used depending on the form of the oil-in-water emulsified cosmetic. For example, there is a method containing blending components (A), (B), and (C), as well as the optional components mentioned above as necessary, and mixing them uniformly with a homomixer or the like.

From the viewpoint of improving the UV protection effect and the durability of the UV protection effect, from the viewpoint of improving the effect of inhibiting the adhesion of air toxic substances, and from the viewpoint of reducing discomfort when wearing a mask, the method for producing the oil-in-water emulsified cosmetic of the present invention is preferably a method containing mixing and emulsifying a dispersion in which the component (A) is dispersed in an oil-phase component containing the components (B) and (C), as well as a component (D) and the oil-soluble optional component mentioned above as necessary, and a preparation of an aqueous-phase component containing an aqueous medium and the water-soluble optional component mentioned above as necessary; and more preferably a method containing the following steps I to III.

In the present invention, the "water-soluble component" as mentioned herein refers to a component which has a uniform appearance when visually observed in a mixture obtained by mixing 100 parts by mass of water and 3 parts by mass of the component at 25°C, and the "oil-soluble component" refers to, as described above, a component which has a non-uniform appearance when visually observed in a mixture obtained by mixing 100 parts by mass of water and 3 parts by mass of the component at 25°C.

Step I: a step of adding and mixing the component (A) with the component (B), the component (C), and optionally the component (D) and the oil-soluble optional component mentioned above to obtain a dispersion (i) in which the component (A) is dispersed

Step II: a step of uniformly mixing an aqueous-phase component containing an aqueous medium and optionally the water-soluble optional component mentioned above to obtain a preparation (ii)

Step III: a step of adding and mixing the dispersion (i) obtained in step I with the preparation (ii) obtained in step II, followed by emulsification, thereby obtaining an oil-in-water emulsified cosmetic

It is preferable to perform mixing in steps I and II by stirring while heating at a temperature in the range of 20°C or higher and 90°C or lower.

In step II, the preparation (ii) is preferably obtained by cooling to a temperature in the range of 15°C or higher and 35°C or lower, followed by uniform mixing.

In step III, it is preferable to add and mix the dispersion (i) obtained in step I preferably kept at 30°C or higher and 90°C or lower, while stirring the preparation (ii) obtained in step II, followed by emulsification.

Regarding the above embodiments, the present invention further discloses the following embodiments.

<1> An oil-in-water emulsified cosmetic comprising the following components (A), (B), and (C):

(A) a fatty acid-coated titanium oxide having a surface tension of 40 mN/m or less;
(B) a volatile hydrocarbon oil having a viscosity at 37.8°C of 7 mPa·s or less (B1) and an ester oil (B2); and
(C) an oil gelling agent,

a content of the component (A) being 3 mass% or more and 20 mass% or less,
a content of the component (B) being 5 mass% or more and 43 mass% or less, and
a mass ratio of the content of the component (B) to a content of an oil-phase component [component (B)/oil-phase component] being 0.50 or more.

<2> The oil-in-water emulsified cosmetic according to <1>, wherein the surface tension of the component (A) is preferably 39 mN/m or less, more preferably 38 mN/m or less, further more preferably 37 mN/m or less, and even more preferably 36 mN/m or less, and is preferably 20 mN/m or more, more preferably 25 mN/m or more, further more preferably 30 mN/m or more, and even more preferably 33 mN/m or more.

<3> The oil-in-water emulsified cosmetic according to <1>, wherein the surface tension of the component (A) is preferably from 20 to 40 mN/m, more preferably from 25 to 39 mN/m, further more preferably from 30 to 38 mN/m, even more preferably from 30 to 37 mN/m, even more preferably from 30 to 36 mN/m, and even more preferably from 33 to 36 mN/m.

<4> The oil-in-water emulsified cosmetic according to any one of <1> to <3>, wherein the component (A) has an average primary particle size of preferably 3 nm or more, more preferably 5 nm or more, and further more preferably 10 nm or more, and of preferably 100 nm or less, more preferably 80 nm or less, further more preferably 50 nm or less, even more preferably 30 nm or less, even more preferably 25 nm or less, and even more preferably 20 nm or less.

<5> The oil-in-water emulsified cosmetic according to any one of <1> to <3>, wherein the component (A) has an average primary particle size of preferably from 3 to 100 nm, more preferably from 5 to 100 nm, further more preferably from 5 to 80 nm, even more preferably from 5 to 50 nm, even more preferably from 5 to 50 nm, even more preferably from 5 to 30 nm or less, even more preferably from 5 to 25 nm, even more preferably from 5 to 20 nm, and even more preferably from 10 to 20 nm.

<6> The oil-in-water emulsified cosmetic according to any one of <1> to <5>, wherein the content of the component (A) is preferably 5 mass% or more, more preferably 8 mass% or more, further more preferably 10 mass% or more, and even more preferably 12 mass% or more, and is preferably 18 mass% or less, more preferably 16 mass% or less, and further more preferably 14 mass% or less.

<7> The oil-in-water emulsified cosmetic according to any one of <1> to <5>, wherein the content of the component (A) is preferably from 3 to 18 mass%, more preferably from 5 to 18 mass%, further more preferably from 8 to 18 mass%, even more preferably from 10 to 18 mass%, even more preferably from 12 to 18 mass%, even more preferably from 12 to 16 mass%, and even more preferably from 12 to 14 mass%.

<8> The oil-in-water emulsified cosmetic according to any one of <1> to <7>, wherein a mass ratio of the content of the component (A) to the content of the oil-phase component in the oil-in-water emulsified cosmetic [component (A)/oil-phase component] is preferably 0.20 or more, more preferably 0.25 or more, further more preferably 0.30 or more, even more preferably 0.35 or more, and even more preferably 0.40 or more, and is preferably 0.80 or less, more preferably 0.70 or less, further more preferably 0.60 or less, and even more preferably 0.50 or less.

<9> The oil-in-water emulsified cosmetic according to any one of <1> to <7>, wherein the mass ratio of the content of the component (A) to the content of the oil-phase component in the oil-in-water emulsified cosmetic [component (A)/oil-phase component] is preferably from 0.20 to 0.80, more preferably from 0.25 to 0.70, further more preferably from 0.30 to 0.60, even more preferably from 0.35 to 0.50, and even more preferably from 0.40 to 0.50.

<10> The oil-in-water emulsified cosmetic according to any one of <1> to <9>, wherein the component (B1) is preferably one or more members selected from the group consisting of a paraffin-based volatile hydrocarbon oil, an isoparaffin-based volatile hydrocarbon oil, and a cyclic paraffin hydrocarbon oil; and more preferably light liquid isoparaffin.

<11> The oil-in-water emulsified cosmetic according to any one of <1> to <10>, wherein the viscosity at 37.8°C of the component (B1) is preferably 6 mPa·s or less, more preferably 5 mPa·s or less, and further more preferably 4 mPa·s or less, and is preferably 0.3 mPa·s or more, more preferably 0.5 mPa·s or more, and further more preferably 1 mPa·s or more.

<12> The oil-in-water emulsified cosmetic according to any one of <1> to <10>, wherein the viscosity at 37.8°C of the component (B1) is preferably from 0.3 to 7 mPa·s, more preferably from 0.5 to 6 mPa·s, further more preferably from 1 to 5 mPa·s, and even more preferably from 1 to 4 mPa·s.

<13> The oil-in-water emulsified cosmetic according to any one of <1> to <12>, wherein the component (B2) is preferably an ester of the formula (1) or the formula (3), more preferably one or more members selected from the group consisting of a monoester of linear or branched fatty acid having 8 or more and 18 or less carbon atoms and linear or branched monohydric alcohol having 2 or more and 24 or less carbon atoms, a triester of branched fatty acid having 6 or more and 18 or less carbon atoms and glycerin, a tetraester of branched fatty acid having 6 or more and 18 or less carbon atoms and pentaerythritol, and a diester of linear or branched fatty acid having 6 or more and 18 or less carbon atoms and branched dihydric alcohol having 2 or more and 10 or less carbon atoms; and more preferably one or more members selected from the group consisting of neopentyl glycol di-2-ethylhexanoate, triethylhexanoin, isotridecyl isononanoate, isononyl isononanoate, and neopentyl glycol dicaprate.

<14> The oil-in-water emulsified cosmetic according to any one of <1> to <13>, wherein the content of the component (B) is preferably 10 mass% or more, more preferably 15 mass% or more, and further more preferably 20 mass% or more, and is preferably 35 mass% or less, more preferably 30 mass% or less, and further more preferably 25 mass% or less.

<15> The oil-in-water emulsified cosmetic according to any one of <1> to <13>, wherein the content of the component (B) is preferably from 10 to 35 mass%, more preferably from 15 to 35 mass%, further more preferably from 15 to 30 mass%, even more preferably from 20 to 30 mass%, and even more preferably from 20 to 25 mass%.

<16> The oil-in-water emulsified cosmetic according to any one of <1> to <15>, wherein the mass ratio of the content of the component (A) to the content of the component (B) [component (A)/component (B)] is preferably 0.20 or more, more preferably 0.30 or more, and further more preferably 0.40 or more, and is preferably 1.0 or less, more preferably 0.90 or less, further more preferably 0.80 or less, even more preferably 0.70 or less, and even more preferably 0.60 or less.

<17> The oil-in-water emulsified cosmetic according to any one of <1> to <15>, wherein the mass ratio of the content of the component (A) to the content of the component (B) [component (A)/component (B)] is preferably from 0.20 to 1.0, more preferably from 0.20 to 0.90, further more preferably from 0.30 to 0.90, even more preferably from 0.40 to 0.80, even more preferably from 0.40 to 0.70, and even more preferably from 0.40 to 0.60.

<18> The oil-in-water emulsified cosmetic according to any one of <1> to <17>, wherein the mass ratio of the content of the component (B) to the content of the oil-phase component in the oil-in-water emulsified cosmetic [component (B)/oil-phase component] is preferably 0.60 or more, more preferably 0.70 or more, further more preferably 0.80 or more, and even more preferably 0.85 or more, and is preferably 1.0 or less, more preferably 0.95 or less, and further more preferably 0.92 or less.

<19> The oil-in-water emulsified cosmetic according to any one of <1> to <17>, wherein the mass ratio of the content of the component (B) to the content of the oil-phase component in the oil-in-water emulsified cosmetic [component (B)/oil-phase component] is preferably from 0.50 to 0.98, more preferably from 0.60 to 0.98, further more preferably from 0.70 to 0.95, even more preferably from 0.80 to 0.95, even more preferably from 0.85 to 0.95, and even more preferably from 0.85 to 0.92.

<20> The oil-in-water emulsified cosmetic according to any one of <1> to <19>, wherein a mass ratio of a content of the component (B1) to a content of the component (B2) [component (B1)/component (B2)] is preferably 0.15 or more, more preferably 0.25 or more, and further more preferably 0.35 or more, and is preferably 3.0 or less, more preferably 2.0 or less, further more preferably 1.7 or less, even more preferably 1.0 or less, even more preferably 0.70 or less, and even more preferably 0.50 or less.

<21> The oil-in-water emulsified cosmetic according to any one of <1> to <19>, wherein the mass ratio of the content of the component (B1) to the content of the component (B2) [component (B1)/component (B2)] is preferably from 0.15 to 3.0, more preferably from 0.25 to 2.0, further more preferably from 0.35 to 1.7, even more preferably from 0.35 to 1.0, even more preferably from 0.35 to 0.70, and even more preferably from 0.35 to 0.50.

<22> The oil-in-water emulsified cosmetic according to any one of <1> to <21>, wherein a mass ratio of the content of the component (B1) to the content of the component (A) [component (B1)/component (A)] is preferably 0.30 or more, more preferably 0.40 or more, and further more preferably 0.50 or more, and is preferably 2.5 or less, more preferably 2.0 or less, further more preferably 1.5 or less, and even more preferably 1.0 or less.

<23> The oil-in-water emulsified cosmetic according to any one of <1> to <21>, wherein the mass ratio of the content of the component (B1) to the content of the component (A) [component (B1)/component (A)] is preferably from 0.30 to 2.5, more preferably from 0.30 to 2.0, further more preferably from 0.30 to 1.5, even more preferably from 0.30 to 1.0, even more preferably 0.40 or more and 1.0 or less, and even more preferably 0.50 or more and 1.0 or less.

<24> The oil-in-water emulsified cosmetic according to any one of <1> to <23>, wherein a mass ratio of the content of the component (B2) to the content of the component (A) [component (B2)/component (A)] is preferably 0.30 or more, more preferably 0.50 or more, further more preferably 0.80 or more, and even more preferably 1.0 or more, and is preferably 1.5 or less.

<25> The oil-in-water emulsified cosmetic according to any one of <1> to <23>, wherein the mass ratio of the content of the component (B2) to the content of the component (A) [component (B2)/component (A)] is preferably from 0.30 to 1.5, more preferably from 0.50 to 1.5, further more preferably from 0.80 to 1.5, and even more preferably from 1.0 to 1.5.

<26> The oil-in-water emulsified cosmetic according to any one of <1> to <25>, wherein the component (C) is one or more members selected from the group consisting of an organically modified clay mineral, a dextrin fatty acid ester, a metal soap, a sucrose fatty acid ester, and an amino acid-based gelling agent.

<27> The oil-in-water emulsified cosmetic according to any one of <1> to <26>, wherein the content of the component (C) is preferably 0.1 mass% or more, more preferably 0.3 mass% or more, further more preferably 0.5 mass% or more, and even more preferably 0.8 mass% or more, and is preferably 3 mass% or less, more preferably 2 mass% or less, further more preferably 1.5 mass% or less, and even more preferably 1.2 mass% or less.

<28> The oil-in-water emulsified cosmetic according to any one of <1> to <26>, wherein the content of the component (C) is preferably from 0.1 to 3 mass%, more preferably from 0.3 to 2 mass%, further more preferably from 0.5 to 1.5 mass%, and even more preferably from 0.8 to 1.2 mass%.

<29> The oil-in-water emulsified cosmetic according to any one of <1> to <28>, wherein a content of a film-forming agent (D) is preferably 0 mass% or more, and is preferably 1 mass% or less, more preferably 0.7 mass% or less, further more preferably 0.4 mass% or less, even more preferably 0.2 mass% or less, and even more preferably 0 mass%.

<30> The oil-in-water emulsified cosmetic according to any one of <1> to <28>, wherein the content of the film-forming agent (D) is preferably from 0 to 1 mass%, more preferably from 0 to 0.7 mass%, further more preferably from 0 to 0.4 mass%, even more preferably from 0 to 0.2 mass%, and even more preferably 0 mass%.

<31> The oil-in-water emulsified cosmetic according to <29> or <30>, wherein the component (D) is one or more members selected from the group consisting of a silicone-based resin, a rosin acid-based resin, a candelilla resin, polyvinyl isobutyl ether, polyisobutylene, and a solid wax (provided that alkyl-modified silicone is excluded).

<32> The oil-in-water emulsified cosmetic according to any one of <1> to <31>, further comprising (E) a surfactant.

<33> The oil-in-water emulsified cosmetic according to <32>, wherein the component (E) is preferably a nonionic surfactant, and more preferably one or more members selected from the group consisting of ceramide, polyhydroxy fatty acid, an ester of pentaerythritol and polyhydroxy fatty acid, a sorbitan fatty acid ester, a polyoxyalkylene sorbitan fatty acid ester, a polyoxyalkylene fatty acid ester, a polyoxyalkylene alkyl ether, a polyoxyalkylene alkenyl ether, an alkyl glucoside, an alkyl glyceryl ether, a sucrose fatty acid ester, a glycerin fatty acid ester, a polyglycerin fatty acid ester, a polyoxyalkylene hydrogenated castor oil, an alkyl saccharide, an alkylamine oxide, an alkyl amidoamine oxide, and polyether-modified silicone.

<34> The oil-in-water emulsified cosmetic according to <32> or <33>, wherein a content of the component (E) is preferably 0.5 mass% or more, more preferably 1 mass% or more, and further more preferably 2 mass% or more, and is preferably 6 mass% or less, more preferably 5 mass% or less, and further more preferably 4 mass% or less.

<35> The oil-in-water emulsified cosmetic according to <32> or <33>, wherein the content of the component (E) is preferably from 0.5 to 6 mass%, more preferably from 1 to 5 mass%, and further more preferably from 2 to 4 mass%.

<36> The oil-in-water emulsified cosmetic according to any one of <32> to <35>, which comprises, as the component (E), a surfactant (E1) in which a calculated value of a common logarithm (clogP) of a partition coefficient P (1-octanol/water) between 1-octanol and water is 10 or more.

<37> The oil-in-water emulsified cosmetic according to <36>, wherein the component (E1) is preferably one or more members selected from the group consisting of N-(hexadecyloxyhydroxypropyl)-N-hydroxyethylhexadecanamide (clogP: 13.4), polyglyceryl diisostearate (clogP: 15.7), sorbitan distearate (clogP: 17.6), dipentaerythrityl tripolyhydroxystearate (clogP: 35.7), and polyhydroxystearic acid (clogP: 34.2); more preferably one or more members selected from the group consisting of dipentaerythrityl tripolyhydroxystearate (clogP: 35.7) and polyhydroxystearic acid (clogP: 34.2); and further more preferably dipentaerythrityl tripolyhydroxystearate (clogP: 35.7).

<38> The oil-in-water emulsified cosmetic according to <36> or <37>, wherein a content of the component (E1) is preferably 0.5 mass% or more, more preferably 1 mass% or more, and further more preferably 2 mass% or more, and is preferably 6 mass% or less, more preferably 5 mass% or less, and further more preferably 4 mass% or less.

<39> The oil-in-water emulsified cosmetic according to <36> or <37>, wherein the content of the component (E1) is preferably from 0.5 to 6 mass%, more preferably from 1 to 5 mass%, and further more preferably from 2 to 4 mass%.

<40> The oil-in-water emulsified cosmetic according to any one of <36> to <39>, wherein when dipentaerythrityl tripolyhydroxystearate (clogP: 35.7) is contained as the component (E1), a mass ratio of a content of dipentaerythrityl tripolyhydroxystearate to the content of the component (A) [dipentaerythrityl tripolyhydroxystearate/component (A)] is preferably 0.01 or more, more preferably 0.02 or more, and further more preferably 0.04 or more, and is preferably 0.15 or less, more preferably 0.10 or less, further more preferably 0.08 or less, and even more preferably 0.07 or less.

<41> The oil-in-water emulsified cosmetic according to any one of <36> to <39>, wherein when dipentaerythrityl tripolyhydroxystearate (clogP: 35.7) is contained as the component (E1), the mass ratio of the content of dipentaerythrityl tripolyhydroxystearate to the content of the component (A) [dipentaerythrityl tripolyhydroxystearate/component (A)] is preferably from 0.01 to 0.15, more preferably from 0.01 to 0.10, further more preferably from 0.02 to 0.08, and even more preferably from 0.04 to 0.07.

<42> The oil-in-water emulsified cosmetic according to any one of <1> to <41>, further comprising (F) a water-soluble polymer.

<43> The oil-in-water emulsified cosmetic according to <42>, wherein the component (F) is preferably one or more members selected from the group consisting of xanthan gum and an acrylic polymer, and preferably xanthan gum from the viewpoint of moisturizing properties, or an acrylic polymer from the viewpoint of emulsion stability; and the acrylic polymer is one or more members selected from the group consisting of (1) crosslinked polyacrylic acid (carbomer), (2) an alkyl-modified carboxyvinyl polymer, (3) polyacrylamide, and (4) a copolymer containing one or more acrylamide-based monomer-derived structural units selected from the group consisting of acrylamide and acryloyldimethyltauric acid.

<44> The oil-in-water emulsified cosmetic according to <42> or <43>, wherein a content of the component (F) is preferably 0.05 mass% or more, more preferably 0.10 mass% or more, further more preferably 0.30 mass% or more, and even more preferably 0.50 mass% or more, and is preferably 3.0 mass% or less, more preferably 2.0 mass% or less, and further more preferably 1.0 mass% or less.

<45> The oil-in-water emulsified cosmetic according to <42> or <43>, wherein the content of the component (F) is preferably from 0.05 to 3.0 mass%, more preferably from 0.10 to 2.0 mass%, further more preferably from 0.30 to 1.0 mass%, and even more preferably from 0.50 to 1.0 mass%.

<46> The oil-in-water emulsified cosmetic according to any one of <1> to <45>, which is for use in sunscreen.

Examples

The present invention will be described in detail below with reference to Examples and Comparative Examples; however, the present invention is not limited thereto.

(Surface tension of component (A))

Measurements were carried out as follows using wetting tension test mixtures having various wetting tensions (manufactured by FUJIFILM Wako Pure Chemical Corporation). The No. in the product name of each mixture indicates the surface tension (mN/m) of the mixture.

In an environment with a temperature of 23°C and a relative humidity of 50%, 20 mL of each wetting tension test mixture was added dropwise to a disposable tray, and 0.01 g of the component (A) to be measured was gently placed thereon. After 10 seconds had elapsed, it was visually confirmed whether the component (A) was wet with the mixture. The state in which the surface of the component (A) is wet with the mixture means that the mixture penetrates the component (A). The state in which the surface of the component (A) is not wet with the mixture means that even when the component (A) and the mixture are mixed, the component (A) is separated (repelled) from the mixture.

The measurement was started with a wetting tension test mixture showing low wetting tension, and the wetting tension of the mixture when the component (A) was no longer wet with the mixture was taken as the surface tension (mN/m) of the component (A).

(Average primary particle size of component (A))

The average primary particle size of the component (A) was measured in the following manner.

In the case of a measurement sample having a shape other than a flaky or plate-like shape, a dispersion of the measurement sample prepared in advance was placed on the sample stage of a transmission electron microscope (TEM) (product name: "JEM 1400 Plus," manufactured by JEOL Ltd.) and air-dried, after which the maximum minor axes of 300 primary particles were measured in an image observed with the TEM at a magnification of 50 000 times, and the number average value thereof was taken as the average primary particle size. The maximum minor axis as mentioned herein refers to, among the minor axes perpendicular to the major axes, a minor axis having the maximum length.

In the case of a measurement sample having a flaky or plate-like shape, the thicknesses of 300 primary particles were measured in an image observed in the same manner and under the same magnification conditions as above, and the number average value thereof was taken as the average primary particle size.

The dispersion of the measurement sample was prepared by adding 95 g of ethanol as a solvent to 5 g of the measurement sample, followed by ultrasonic dispersion.

(Viscosity of component (B1))

The viscosity of the component (B1) was measured with a B-type viscometer (model name: "TVB10M," manufactured by Toki Sangyo Co., Ltd.) using rotor No. 1 at a rotational speed of 60 rpm and a temperature of 37.8°C for 1 minute.

Examples 1 to 6 and Comparative Examples 1 to 6

Oil-in-water emulsified cosmetics were each obtained in the following manner in accordance with the formulations shown in Tables 1 and 2.

The amount of each of the components shown in Tables 1 and 2 is the effective amount.

The components 6, 8 to 18, and 23 to 26 shown in Tables 1 and 2 were mixed and dissolved by heating to 90°C to obtain a preparation (i'). Then, while keeping the resulting preparation (i') at 70°C, the components 1 to 5 shown in Tables 1 and 2 were added and mixed, and the resulting mixture was dispersed using a disperser at 70°C and 5 000 rpm for 20 minutes, thereby obtaining a dispersion (i) (step I).

Separately, the components 22 and 29 to 33 shown in Tables 1 and 2 were dispersed using a disperser at 50°C and 1 500 rpm for 10 minutes, after which the components 27 and 28 were added and mixed using a homomixer at 50°C and 8 000 rpm for 1 minute. Then, the components 7 and 19 to 21 were added and mixed using a homomixer at 50°C and 8 000 rpm for 1 minute, thereby obtaining a preparation (ii) (step II).

The dispersion (i) was added while stirring the resulting preparation (ii) using a homomixer at 50°C and 8 000 rpm, after which the resulting mixture was cooled to 25°C while stirring at 200 rpm using propeller blades, thereby obtaining an oil-in-water emulsified cosmetic (step III).

The resulting oil-in-water emulsified cosmetics were evaluated for the following: (1) UV protection ability, (2) durability of UV protection ability, (3) effect of inhibiting the adhesion of air toxic substances, (4) discomfort when wearing a mask, and (5) use impression during application. The results are shown in Tables 1 and 2.

(1) UV protection ability

Each oil-in-water emulsified cosmetic was uniformly applied to a square PMMA plate so that the application amount was 1.3 mg/cm$^2$, and then dried in a cool, dark place for 15 minutes. After drying, the transmittance (%) of the absorption spectrum (wavelength: 350 nm) was measured at a total of 9 points on the PMMA plate, namely the midpoint, each vertex, and the midpoint of the side connecting each vertex, using an SPF analyzer (SPF Analyzer UV-2000S, manufactured by Labsphere), and the average transmittance of the nine points was determined. The SPF value was calculated from the average transmittance (%), and the UV protection ability was evaluated based on the following criteria.

(Evaluation criteria)

5: The SPF value was 30 or more.
4: The SPF value was 25 or more and less than 30.
3: The SPF value was 20 or more and less than 25.
2: The SPF value was 15 or more and less than 20.
1: The SPF value was less than 15.

(2) Durability of UV protection ability

Three female panelists were asked to apply 0.2 g of each oil-in-water emulsified cosmetic to the entire face. After drying for 15 minutes, UV protection efficacy (UVPE) was determined using a multispectral UV polarization reflectance imaging system (MUPRIS) in accordance with the method described in the document (Nishino et al., Skin Research and Technology, 2019, Volume 25, Issue 5, pp. 639-652). The UVPE at this time (i.e., the UVPE after drying for 15 minutes) was taken as the initial value.
Thereafter, the UVPE after 6 hours of daily life with a mask on was similarly determined using the multispectral UV polarization reflectance imaging system (MUPRIS).
The initial UVPE was set to 1, and the UV protection ability duration rate was calculated from the UVPE after 6 hours and evaluated based on the following criteria.

(Evaluation criteria)

5: The UV protection ability duration rate was 92.5% or more.
4: The UV protection ability duration rate was 85% or more and less than 92.5%.
3: The UV protection ability duration rate was 77.5% or more and less than 85%.
2: The UV protection ability duration rate was 70% or more and less than 77.5%.
1: The UV protection ability duration rate was less than 70%.

(3) Effect of inhibiting the adhesion of air toxic substances

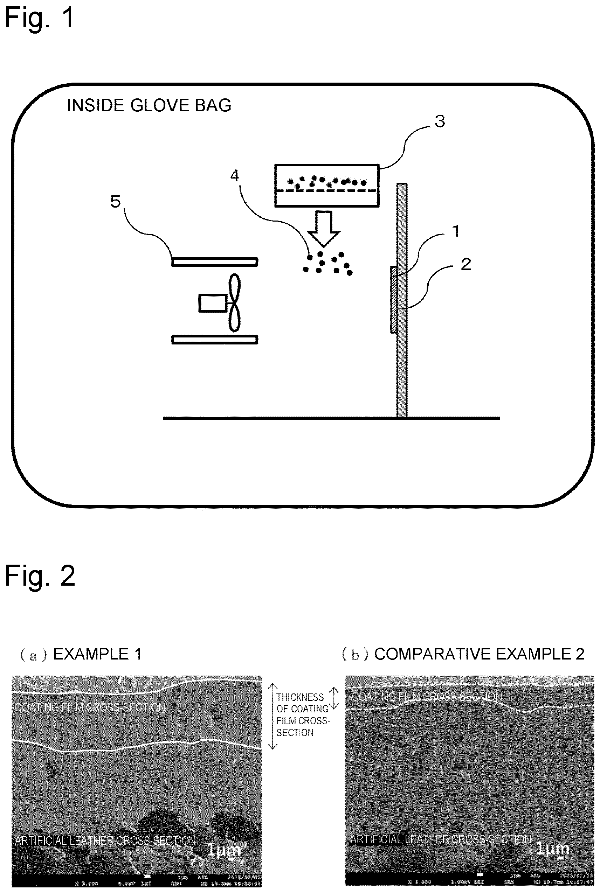

Each oil-in-water emulsified cosmetic was applied to white artificial leather (product name: "Laforet S2923," manufactured by Okamoto Shinwa Co., Ltd.) as a skin replacement so that the amount of the component (A) applied was 0.12 mg/cm$^2$, and the artificial leather was left to dry overnight at room temperature to obtain a test sample.
The surface of the artificial leather coated with each oil-in-water emulsified cosmetic was exposed to an environment in which an air toxic substance for evaluation was blown, and the color difference ΔE before and after exposure was measured using a colorimeter in the following manner.
Using a colorimeter (product name: "CM-2002," manufactured by Konica Minolta, Inc.), the $L^*_1$, $a^*_1$, and $b^*_1$ values of the surface of the artificial leather as the test sample before exposure were measured.
Separately, a blower (product name: "Silky Wind 9ZF002RH02," size: 129 × 106 × 83 mm, manufactured by Rhythm Watch Co., Ltd.) and a mesh sieve (Test sieve JIS Z 8801, frame dimensions: φ100 × 45H, mesh size: 106 μm, manufactured by Tokyo Screen Co., Ltd.) were fixed into a glove bag (product number: "3-118-01," manufactured by AS ONE Corporation). The mesh sieve was set at a height of 17 cm.
Next, the artificial leather (5 cm × 4 cm) coated with each oil-in-water emulsified cosmetic as the test sample was attached to a support so that the bottom end of the artificial leather was at a height of 11 cm. The distance between the coated surface of the artificial leather attached to the support and the blower was set to 15 cm, and as shown in Fig. 1, the blower was set so that the coated surface of the artificial leather was perpendicular to the blowing direction of the blower, and so that the center height of the blower blades was the same as the center height of the artificial leather.
The temperature inside the glove bag was set to 25°C and the relative humidity to 57% RH, and 50 mg of graphite powder (product name: "J-CPB," average particle size: 5.5 μm, manufactured by Nippon Kokuen Group) as the air toxic substance for evaluation was dropped through the mesh sieve for 1 minute in front of the outlet of the blower with a blowing scale set to 1, while being classified using a clogging removal brush (product name: "JNB-5," brush diameter: 53 μm, manufactured by Tokyo Screen Co., Ltd.), thereby exposing the surface of the artificial leather coated with each cosmetic to the environment in which the air toxic substance for evaluation was blown.
Next, the $L^*_2$, $a^*_2$, and $b^*_2$ values of the surface of the artificial leather as the test sample after exposure were measured using the colorimeter mentioned above, and the color difference ΔE value was calculated by the following formula (I).

$$\Delta E \ = \ [\,(L^{*}_{1}-L^{*}_{2})^{2}+(a^{*}_{1}-a^{*}_{2})^{2}+(b^{*}_{1}-b^{*}_{2})^{2}\,]^{0.5} \quad (I)$$

The above procedure was carried out three times for each test sample, and the average value of the color difference ΔE of the artificial leather coated with each oil-in-water emulsified cosmetic as the test sample was taken as ΔEt. Further, as a standard sample, the same procedure as above was also carried out three times on artificial leather coated with no oil-in-water emulsified cosmetic, the average value of the color difference ΔE was taken as ΔEs, and the adhesion inhibition rate was calculated by the following formula (II) and evaluated based on the following criteria. The higher the adhesion inhibition rate is, the better the effect of inhibiting the adhesion of air toxic substances is. Here, L*, a*, and b* refer to L*, a*, b* specified by the CIE 1976 L*a*b* color system, respectively.

Air toxic substance adhesion inhibition rate (%) = 100 × (ΔEs-ΔEt)/ΔEs (II)                    (II)

(Evaluation criteria)

5: The adhesion inhibition rate was 50% or more.
4: The adhesion inhibition rate was 40% or more and less than 50%.
3: The adhesion inhibition rate was 20% or more and less than 40%.
2: The adhesion inhibition rate was 10% or more and less than 20%.
1: The adhesion inhibition rate was less than 10%.

(4) Discomfort when wearing mask

Five expert panelists were asked to apply 1 mg of each oil-in-water emulsified cosmetic to the entire face. After drying for 15 minutes, the panelists were asked to wear a mask and then continue to wear the mask for 6 hours. The panelists were asked to rate the presence or absence of discomfort caused by the mask based on the following criteria, and the average scores of the five expert panelists were calculated.

(Scores and evaluation criteria)

5: Did not feel any discomfort due to the mask
4: Hardly felt discomfort due to the mask
3: Neither
2: Slightly felt discomfort due to the mask
1: Strongly felt discomfort due to the mask

(5) Use impression during application

Five expert panelists were asked to apply 1 mg of each oil-in-water emulsified cosmetic to the entire face. At this time, the panelists were asked to rate the use impression during application (spreading during application and skin compatibility) based on the following criteria, and the average scores of the five expert panelists were calculated.

(Scores and evaluation criteria)

5: Good spreading and skin compatibility
4: Slightly good spreading and skin compatibility
3: Neither
2: Slightly poor spreading and skin compatibility
1: Poor spreading and skin compatibility

For the evaluation items (4) and (5), each sample was evaluated on a five-point scale from A to E based on the average evaluation score.

A: The average evaluation score was 4.6 or more and 5.0 or less.
B: The average evaluation score was 3.6 or more and less than 4.6.
C: The average evaluation score was 2.6 or more and less than 3.6.
D: The average evaluation score was 1.6 or more and less than 2.6.
E: The average evaluation score was 1.0 or more and less than 1.6.

[Table 1]

Table 1

| No. | Component | | Type | Example | | | | | |
|---|---|---|---|---|---|---|---|---|---|
| | | | | 1 | 2 | 3 | 4 | 5 | 6 |
| 1 | Component (A) | | Aluminum hydroxide/stearic acid-coated titanium oxide microparticles A1 *1 | 12.0 | | | | | 12.0 |
| 2 | | | Aluminum hydroxide/stearic acid-coated titanium oxide microparticles A2 *2 | | 12.0 | | | | |
| 3 | | | Aluminum hydroxide/stearic acid-coated titanium oxide microparticles A3 *3 | | | 12.0 | | | |
| 4 | | | Aluminum hydroxide/stearic acid-coated titanium oxide microparticles A4 *4 | | | | 12.0 | | |
| 5 | Component (A') | | Aluminum hydroxide/stearic acid-coated titanium oxide microparticles A'1 *5 | | | | | | |
| 6 | Component (B) | Component (B1) | Light liquid isoparaffin *6 | 6.0 | 6.0 | 6.0 | 6.0 | 6.0 | 25.0 |
| 7 | | | Isohexadecane *18 | 0.34 | 0.34 | 0.34 | 0.34 | 0.34 | 0.34 |
| 8 | | Componen (B2) | Neopentyl glycol di-2-ethylhexanoate *7 | 2.7 | 2.7 | 2.7 | 2.7 | 2.7 | 2.7 |
| 9 | | | Triethylhexanoin *8 | 2.8 | 2.8 | 2.8 | 2.8 | 2.8 | 2.8 |
| 10 | | | Isotridecyl isononanoate *9 | 3.0 | 3.0 | 3.0 | 3.0 | 3.0 | 3.0 |
| 11 | | | Isononyl isononanoate *10 | 5.4 | 5.4 | 5.4 | 5.4 | 5.4 | 5.4 |
| 12 | | | Neopentyl glycol dicaprate *11 | 3.0 | 3.0 | 3.0 | 3.0 | 3.0 | 3.0 |
| 13 | Component (C) | | Dextrin palmitate *12 | 1.0 | 1.0 | 1.0 | 1.0 | 1.0 | 1.0 |

| Component | | | | Example | | | | | |
|---|---|---|---|---|---|---|---|---|---|
| | No. | | Type | 1 | 2 | 3 | 4 | 5 | 6 |
| Blending of oil-in-water emulsified cosmetic (mass%) | 14 | Component (E) | Component (E1) | N-(hexadecyloxyhydroxypropyl) -N-hydroxyethylhexadecanamide *13 | 1.3 | 1.3 | 1.3 | 1.3 | 1.3 | 1.3 |
| | 15 | | | Dipentaerythrityl tripolyhydroxystearate*14 | 0.6 | 0.6 | 0.6 | 0.6 | 0.6 | 0.6 |
| | 16 | | | Polyhydroxystearic acid *15 | 0.1 | 0.1 | 0.1 | 0.1 | 0.1 | 0.1 |
| | 17 | | | Polyalyceryl diisostearate *16 | 0.5 | 0.5 | 0.5 | 0.5 | 0.5 | 0.5 |
| | 18 | | | Sorbitan distearate *17 | 0.3 | 0.3 | 0.3 | 0.3 | 0.3 | 0.3 |
| | 19 | | Polysorbate 80 *18 | | 0.11 | 0.11 | 0.11 | 0.11 | 0.11 | 0.11 |
| | 20 | | Sorbitan oleate *18 | | 0.04 | 0.04 | 0.04 | 0.04 | 0.04 | 0.04 |
| | 21 | Component (F) | Sodium acrylate/sodium acryloyldimethyl taurate copolymer *18 | | 0.56 | 0.56 | 0.56 | 0.56 | 0.56 | 0.56 |
| | 22 | | Alkyl acrylate/methacrylate copolymer *19 | | 0.2 | 0.2 | 0.2 | 0.2 | 0.2 | 0.2 |
| | 23 | Component (G) | Squalane *20 | | | | | | | |
| | 24 | | Octamethyltrisiloxane *21 | | 2.0 | 2.0 | 2.0 | 2.0 | 2.0 | 2.0 |
| | 25 | | Cetyl alcohol | | 0.3 | 0.3 | 0.3 | 0.3 | 0.3 | 0.3 |
| | 26 | | Stearyl alcohol | | 0.2 | 0.2 | 0.2 | 0.2 | 0.2 | 0.2 |
| | 27 | Potassium hydroxide aqueous solution *22 | | | 0.18 | 0.18 | 0.18 | 0.18 | 0.18 | 0.18 |
| | 28 | 2-Amino-2-hydroxymethyl-1,3-propanediol *23 | | | 0.1 | 0.1 | 0.1 | 0.1 | 0.1 | 0.1 |
| | 29 | 1,3-Butylene glycol | | | 4.0 | 4.0 | 4.0 | 4.0 | 4.0 | 4.0 |
| | 30 | Dipropylene glycol | | | 3.0 | 3.0 | 3.0 | 3.0 | 3.0 | 3.0 |
| | 31 | Disodium edetate | | | 0.03 | 0.03 | 0.03 | 0.03 | 0.03 | 0.03 |
| | 32 | Phenoxyethanol | | | 0.4 | 0.4 | 0.4 | 0.4 | 0.4 | 0.4 |
| | 33 | Water | | | Balance | Balance | Balance | Balance | Balance | Balance |
| Total | | | | | 100 | 100 | 100 | 100 | 100 | 100 |
| Su rface tension of component (A) | | | | | 35 | 37 | 37 | 38 | 35 | 35 |

EP 4 670 707 A1

| | Component | | Example | | | | | |
|---|---|---|---|---|---|---|---|---|
| | No. | Type | 1 | 2 | 3 | 4 | 5 | 6 |
| | | Con tent of comp onent (A) (mass%) | 12.0 | 12.0 | 12.0 | 12.0 | 18.0 | 12.0 |
| | | Con of comp onent (B) (mass%) | 23.24 | 23.24 | 23.24 | 23.24 | 23.24 | 42.24 |
| | | Mass ratio in oil-in-water emulsified cosmetic [component (A)/component (B)] | 0.52 | 0.52 | 0.52 | 0.52 | 0.77 | 0.28 |
| | | Content of oil-phase component (mass%) | 26.74 | 26.74 | 26.74 | 26.74 | 26.74 | 45.74 |
| | | Mass in oil-in-water emulsified com cosmetic [component (A)/oil-phase ponent] | 0.45 | 0.45 | 0.45 | 0.45 | 0.67 | 0.26 |
| | | Mass in oil-in-water emulsified com cosmetic [component (B)/oil-phase ponent] | 0.87 | 0.87 | 0.87 | 0.87 | 0.87 | 0.92 |
| | | Mass ratico in oil-in-water emulsified cosmetic [component (B1)/component (A)] | 0.53 | 0.53 | 0.53 | 0.53 | 0.35 | 2.11 |
| | | Mass ratio in oil-in-water emulsified cosmetic [component (B2)/component (A)] | 1.41 | 1.41 | 1.41 | 1.41 | 0.94 | 1.41 |
| | | Mass ratio [dipentaerythr in oil-in-water rityl tripolyhy emulsified cosmetic droxystearate/component (A)] | 0.05 | 0.05 | 0.05 | 0.05 | 0.03 | 0.05 |
| Evaluation | | UV protection ability | 5 | 4 | 4 | 3 | 5 | 5 |
| | | Durability of UV protection ability | 5 | 4 | 4 | 4 | 5 | 5 |
| | | Effect of inhibiting adhesion of air toxic substances | 5 | 5 | 5 | 5 | 5 | 3 |
| | | Discomfort when wearina mask | A | A | A | A | A | C |
| | | Use impression ession during application | A | A | A | A | B | A |

EP 4 670 707 A1

26

[Table 2]

[Table 2]

Table 2

| Component | | | | Comparative Example | | | | | |
|---|---|---|---|---|---|---|---|---|---|
| No.1 | | | Type | 1 | 2 | 3 | 4 | 5 | 6 |
| 1 | Component (A) | | Aluminum hydroxide/stearic acid-coated titanium oxide microparticles A1 *1 | 2.0 | | 12.0 | 12.0 | 12.0 | 12.0 |
| 2 | | | Aluminum hydroxide/stearic acid-coated titanium oxide microparticles A2 *2 | | | | | | |
| 3 | | | Aluminum hydroxide/stearic acid-coated titanium oxide microparticles A3 *3 | | | | | | |
| 4 | | | Aluminum hydroxide/stearic acid-coated titanium oxide microparticles A4 *4 | | | | | | |
| 5 | Component (A') | | Aluminum hydroxide/stearic acid-coated titanium oxide microparticles A'1 *5 | | 12.0 | | | | |
| 6 | Component (B) | Component Light (B1) | liquid isoparaffin *6 | 6.0 | 6.0 | | 1.3 | 30.0 | 30.0 |
| 7 | | | Isohexadecane *18 | 0.34 | 0.34 | 0.34 | 0.34 | 0.34 | 0.34 |
| 8 | | Component (B2) | Neopentyl alycol di-2-ethylhexanoate *7 | 2.7 | 2.7 | | 0.6 | 2.7 | 2.7 |
| 9 | | | Triethylhexanoin *8 | 2.8 | 2.8 | | 0.6 | 2.8 | 2.8 |
| 10 | | | Isotridecyl isononanoate *9 | 3.0 | 3.0 | | 0.6 | 3.0 | 3.0 |
| 11 | | | Isononyl isononanoate *10 | 5.4 | 5.4 | | 1.1 | 5.4 | 5.4 |
| 12 | | | Neopentyl alycol dicaprate *11 | 3.0 | 3.0 | | 0.6 | 3.0 | 3.0 |
| 13 | Component (C) | | Dextrin palmitate *12 | 1.0 | 1.0 | 1.0 | 1.0 | 1.0 | |
| 14 | Component (E) | Component (E1) | N-(hexadecyloxyhydroxypropyl) -N-hydroxyethylhexadecanamide *13 | 1.3 | 1.3 | 1.3 | 1.3 | 1.3 | 1.3 |
| 15 | | | Dipentaerythrityl tripolyhydroxystearate*14 | 0.6 | 0.6 | 0.6 | 0.6 | 0.6 | 0.6 |
| 16 | | | Polyhydroxvstearic acid *15 | 0.1 | 0.1 | 0.1 | 0.1 | 0.1 | 0.1 |

Blending of oil-in-water emulsified cosmetic (mass%)

| Component | | | | Co mparative Exam ple | | | | | |
|---|---|---|---|---|---|---|---|---|---|
| No.1 | | Type | | 1 | 2 | 3 | 4 | 5 | 6 |
| 17 | | | Polyalyceryl diisostearate *16 | 0.5 | 0.5 | 0.5 | 0.5 | 0.5 | 0.5 |
| 18 | | | Sorbitan distearate *17 | 0.3 | 0.3 | 0.3 | 0.3 | 0.3 | 0.3 |
| 19 | | Polysorbate 80 *18 | | 0.11 | 0.11 | 0.11 | 0.11 | 0.11 | 0.11 |
| 20 | | Sorbitan oleate *18 | | 0.04 | 0.04 | 0.04 | 0.04 | 0.04 | 0.04 |
| 21 | Component (F) | Sodium acrylate/sodium acryloyldimethyl taurate copolymer *18 | | 0.56 | 0.56 | 0.56 | 0.56 | 0.56 | 0.56 |
| 22 | | Alkyl acrylate/methacrylate copolymer *19 | | 0.2 | 0.2 | 0.2 | 0.2 | 0.2 | 0.2 |
| 23 | Component (G) | Squalane *20 | | | | 22.9 | | | |
| 24 | | Octamethyltrisiloxane *21 | | 2.0 | 2.0 | 2.0 | 8.0 | 2.0 | 2.0 |
| 25 | | Cetyl alcohol | | 0.3 | 0.3 | 0.3 | 0.3 | 0.3 | 0.3 |
| 26 | | Stearyl alcohol | | 0.2 | 0.2 | 0.2 | 0.2 | 0.2 | 0.2 |
| 27 | Potassium hydroxide aqueous solution *22 | | | 0.18 | 0.18 | 0.18 | 0.18 | 0.18 | 0.18 |
| 28 | 2-Amino-2-hydroxymethyl-1,3-propanediol *23 | | | 0.1 | 0.1 | 0.1 | 0.1 | 0.1 | 0.1 |
| 29 | 1,3-Butylene glycol | | | 4.0 | 4.0 | 4.0 | 4.0 | 4.0 | 4.0 |
| 30 | Dipropylene glycol | | | 3.0 | 3.0 | 3.0 | 3.0 | 3.0 | 3.0 |
| 31 | Disodium edetate | | | 0.03 | 0.03 | 0.03 | 0.03 | 0.03 | 0.03 |
| 32 | Phenoxyethanol | | | 0.4 | 0.4 | 0.4 | 0.4 | 0.4 | 0.4 |
| 33 | Water | | | Balance | Balance | Balance | Balance | Balance | Balance |
| Total | | | | 100 | 100 | 100 | 100 | 100 | 100 |
| Surface tension of component (A) | | | | 35 | 42 | 35 | 35 | 35 | 35 |
| Content of component (A) (mass%) | | | | 2.0 | 0.0 | 12.0 | 12.0 | 12.0 | 12.0 |
| Content of component (B) (mass%) | | | | 23.24 | 23.24 | 0.34 | 5.15 | 47.24 | 47.24 |
| Mass ratio in oil-in-water emulsified cosmetic [component (A)/component (B)] | | | | 0.09 | 0.00 | 35.29 | 2.33 | 0.25 | 0.25 |
| Content of oil-phase component (mass%) | | | | 26.74 | 26.74 | 26.74 | 13.65 | 50.74 | 49.74 |
| Mass ratio in oil-in-water emulsified cosmetic [component (A)/oil-phase component] | | | | 0.07 | 0.00 | 0.45 | 0.88 | 0.24 | 0.24 |
| Mass ratio in oil-in-water emulsified cosmetic [component (B)/oil-phase component] | | | | 0.87 | 0.87 | 0.01 | 0.38 | 0.93 | 0.95 |

| | Component | Comparative Example | | | | | |
|---|---|---|---|---|---|---|---|
| No.1 | Type | 1 | 2 | 3 | 4 | 5 | 6 |
| Mass ratio in oil-in-water emulsified cosmetic [component (B1)/component (A)] | | 3.17 | | 0.03 | 0.13 | 2.53 | 2.53 |
| Mass ratio in oil-in-water emulsified cosmetic [component (B2)/component (A)] | | 8.45 | | 0.00 | 0.30 | 1.41 | 1.41 |
| Mass ratio in oil-in-water emulsified cosmetic [dipentaerythrityl tripolyhydroxystearate/component (A)] | | 0.30 | | 0.05 | 0.05 | 0.05 | 0.05 |
| Evaluation | UV protection ability | 2 | 1 | 2 | 1 | 5 | 2 |
| | Durability of UV protection ability | 2 | 1 | 2 | 1 | 5 | 2 |
| | Effect of inhibiting adhesion of air toxic substances | 2 | 3 | 1 | 2 | 1 | 2 |
| | Discomfort when wearing mask | D | C | D | E | D | E |

(continued)

| | Component | | Comparative Example | | | | | |
|---|---|---|---|---|---|---|---|---|
| No.1 | Type | | 1 | 2 | 3 | 4 | 5 | 6 |
| | Use impression during application | | A | C | B | A | D | D |

The details of the components used in Tables 1 and 2 are shown below.

*1: "MPT-171," manufactured by Ishihara Sangyo Kaisha, Ltd. (surface tension: 35 mN/m, average primary particle size: 15 nm, titanium oxide ($TiO_2$) content: 81.0 mass%, stearic acid coating amount: 8.5 mass%) *2: "STR-100C-LF," manufactured by Sakai Chemical Industry Co., Ltd. (surface tension: 37 mN/m, average primary particle size: 10 nm, titanium oxide ($TiO_2$) content: 84.0 mass%, stearic acid coating amount: 7.0 mass%) *3: "MT-N1," manufactured by Tayca Corporation (surface tension: 37 mN/m, average primary particle size: 10 nm or less, titanium oxide ($TiO_2$) content: 66.7 mass%, stearic acid coating amount: 20.0 mass%) *4: "MT-01," manufactured by Tayca Corporation (surface tension: 38 mN/m, average primary particle size: 10 nm, titanium oxide ($TiO_2$) content: 73.0 mass%, stearic acid coating amount: 11.0 mass%) *5: "MT-100TV," manufactured by Tayca Corporation (surface tension: 42 mN/m, average primary particle size: 15 nm, titanium oxide ($TiO_2$) content: 83 mass%, stearic acid coating amount: 8.0 mass%) *6: "Parleam 4," manufactured by NOF Corporation (viscosity at 37.8°C: 3.8 mPa·s) *7: "Cosmol 525," manufactured by Nisshin OilliO Group, Ltd.

*8: "T.I.O," manufactured by Nisshin OilliO Group, Ltd.

*9: "Salacos 913," manufactured by Nisshin OilliO Group, Ltd.

*10: "Salacos 99," manufactured by Nisshin OilliO Group, Ltd.

*11: "Estemol N-01," manufactured by Nisshin OilliO Group, Ltd.

*12: "Rheopearl KL2," manufactured by Chiba Flour Milling Co., Ltd.

*13: "Sphingolipid E," manufactured by Kao Corporation *14: "Salacos WO-6," manufactured by Nisshin OilliO Group, Ltd.

*15: "Salacos HS-6C," manufactured by Nisshin OilliO Group, Ltd.

*16: "Cosmol 42V," manufactured by Nisshin OilliO Group, Ltd.

*17: "Sunsoft NO. 63C-C," manufactured by Taiyo Kagaku Co., Ltd.

*18: "SIMULGEL EG QD," manufactured by SEPPIC S.A. ((sodium acrylate/sodium acryloyldimethyl taurate) copolymer/isohexadecane/Polysorbate 80/sorbitan oleate/water, content of sodium acrylate/sodium acryloyldimethyl taurate copolymer: 37.5 mass%) *19: "PEMULEN TR-2 POLYMER," manufactured by Lubrizol Advanced Materials *20: manufactured by Nippon Surfactant Industries Co., Ltd.

*21: "KF-96A-1CS," manufactured by Shin-Etsu Chemical Co., Ltd.

*22: "48% Liquid Caustic Potash," manufactured by U.S. Occidental Chemical Corporation *23: "TRIS AMINO ULTRA PC," manufactured by Angus Chemical Company

Tables 1 and 2 reveal that compared with the oil-in-water emulsified cosmetics of the Comparative Examples, the oil-in-water emulsified cosmetics of the Examples of the present invention have better use impression during application, have a more excellent UV protection effect and a more excellent effect of inhibiting the adhesion of air toxic substances, have durability of the UV protection effect, and further can form cosmetic coating films which can reduce discomfort when wearing a mask.

In addition, each of the oil-in-water emulsified cosmetics of the Examples and Comparative Examples was applied to the surface of artificial leather ($2\,mg/cm^2$) and dried for 30 minutes to form a coating film. The coating film was cut, the cross-section was observed at a magnification of 3 000 times using a scanning electron microscope (Cryo-SEM (Cryosystem: PP3010T, manufactured by Quorum Technologies, SEM: JEM-7600F, manufactured by JEOL Ltd.)), and nine electron micrographs were obtained. Some of them are shown in Fig. 2. Fig. 2 (a) shows a cross-section of the coating film of Example 1, and (b) shows a cross-section of the coating film of Comparative Example 2, with the boundaries of the coating film being indicated by white solid lines in (a) and by white dashed lines in (b). From the nine electron micrographs obtained, the film thickness of each example was calculated from the average value of 63 points ($9 \times 7$ points) measured on the ridge of the coating film at 7 points spaced 5 μm apart from each other for each micrograph. The results were 8.20 μm ($\pm 2.09$ μm) for Example 1 and 3.84 μm ($\pm 2.78$ μm) for Comparative Example 2 (the numbers in brackets indicate standard deviations). Therefore, the oil-in-water emulsified cosmetic of the present invention is considered to be able to increase the thickness of the cosmetic coating film formed on the skin, thereby providing good use impression during application, more effectively exhibiting a UV protection effect and an effect of inhibiting the adhesion of air toxic substances, and further improving the durability of the UV protection effect. In addition, it is considered that when wearing a mask, irritation to the skin caused by the mask fibers can be further reduced, thereby reducing discomfort when wearing the mask.

Reference Signs List

1: Sample to be evaluated
2: Support
3: Mesh sieve
4: Air toxic substance for evaluation
5: Blower

**Claims**

1.  An oil-in-water emulsified cosmetic comprising the following components (A), (B), and (C):

    (A) a fatty acid-coated titanium oxide having a surface tension of 40 mN/m or less;
    (B) a volatile hydrocarbon oil having a viscosity at 37.8°C of 7 mPa·s or less (B1) and an ester oil (B2); and
    (C) an oil gelling agent,
    a content of the component (A) being 3 mass% or more and 20 mass% or less,
    a content of the component (B) being 5 mass% or more and 43 mass% or less, and
    a mass ratio of the content of the component (B) to a content of an oil-phase component [component (B)/oil-phase component] being 0.50 or more.

2.  The oil-in-water emulsified cosmetic according to claim 1, wherein the component (C) is one or more members selected from the group consisting of an organically modified clay mineral, a dextrin fatty acid ester, a metal soap, a sucrose fatty acid ester, and an amino acid-based gelling agent.

3.  The oil-in-water emulsified cosmetic according to claim 1 or 2, wherein a content of the component (C) is 0.1 mass% or more and 3 mass% or less.

4.  The oil-in-water emulsified cosmetic according to any one of claims 1 to 3, wherein a mass ratio of the content of the component (A) to the content of the component (B) [component (A)/component (B)] is 0.20 or more and 1.0 or less.

5.  The oil-in-water emulsified cosmetic according to any one of claims 1 to 4, wherein a mass ratio of a content of the component (B1) to the content of the component (A) [component (B1)/component (A)] is 0.30 or more and 2.5 or less.

6. The oil-in-water emulsified cosmetic according to any one of claims 1 to 5, wherein a mass ratio of a content of the component (B2) to the content of the component (A) [component (B2)/component (A)] is 0.30 or more and 1.5 or less.

7. The oil-in-water emulsified cosmetic according to any one of claims 1 to 6, wherein a mass ratio of the content of the component (A) to the content of the oil-phase component in the oil-in-water emulsified cosmetic [component (A)/oil-phase component] is 0.20 or more and 0.80 or less.

8. The oil-in-water emulsified cosmetic according to any one of claims 1 to 7, wherein a content of a film-forming agent (D) is 0 mass% or more and 1 mass% or less.

9. The oil-in-water emulsified cosmetic according to any one of claims 1 to 8, which is for use in sunscreen.

# Fig. 1

INSIDE GLOVE BAG

3

4

5

1

2

# Fig. 2

(a) EXAMPLE 1

(b) COMPARATIVE EXAMPLE 2

COATING FILM CROSS-SECTION

ARTIFICIAL LEATHER CROSS-SECTION

1μm

THICKNESS
OF COATING
FILM CROSS-
SECTION

COATING FILM CROSS-SECTION

ARTIFICIAL LEATHER CROSS-SECTION

1μm

EP 4 670 707 A1

## INTERNATIONAL SEARCH REPORT

| International application No. |
| --- |
| **PCT/JP2024/006147** |

**A.    CLASSIFICATION OF SUBJECT MATTER**

*A61K 8/29*(2006.01)i; *A61K 8/04*(2006.01)i; *A61K 8/31*(2006.01)i; *A61K 8/37*(2006.01)i; *A61K 8/73*(2006.01)i; *A61Q 17/04*(2006.01)i

FI:    A61K8/29; A61K8/04; A61K8/31; A61K8/37; A61K8/73; A61Q17/04

According to International Patent Classification (IPC) or to both national classification and IPC

**B.    FIELDS SEARCHED**

Minimum documentation searched (classification system followed by classification symbols)

A61K8/29; A61K8/04; A61K8/31; A61K8/37; A61K8/73; A61Q17/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2020-200316 A (KAO CORPORATION) 17 December 2020 (2020-12-17) paragraphs [0005], [0012], [0013], [0016], [0037]-[0039], [0071], [0105], [0111], [0113] | 1-9 |
| Y | JP 2017-197434 A (KAO CORPORATION) 02 November 2017 (2017-11-02) claims 1, 5, paragraphs [0002], [0005], [0010], [0033], [0034] | 1-9 |
| A | JP 2022-117486 A (KAO CORPORATION) 10 August 2022 (2022-08-10) entire text | 1-9 |
| A | JP 2016-94405 A (KAO CORPORATION) 26 May 2016 (2016-05-26) entire text | 1-9 |
| A | JP 2014-105207 A (ISEHAN K.K.) 09 June 2014 (2014-06-09) entire text | 1-9 |
| A | JP 2020-55806 A (KAO CORPORATION) 09 April 2020 (2020-04-09) entire text | 1-9 |

☐ Further documents are listed in the continuation of Box C.         ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **15 April 2024** | **07 May 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

# EP 4 670 707 A1

## INTERNATIONAL SEARCH REPORT
### Information on patent family members

International application No.

**PCT/JP2024/006147**

| Patent document cited in search report | | | Publication date (day/month/year) | Patent family member(s) | | | Publication date (day/month/year) |
|---|---|---|---|---|---|---|---|
| JP | 2020-200316 | A | 17 December 2020 | US 2022/0249339 A1 paragraphs [0016], [0017], [0041]-[0044], [0054], [0055], [0068]-[0077], [0212]-[0215], [0329], [0522]-[0559] WO 2020/246556 A1 EP 3981384 A1 KR 10-2022-0004186 A CN 113939265 A | | | |
| JP | 2017-197434 | A | 02 November 2017 | (Family: none) | | | |
| JP | 2022-117486 | A | 10 August 2022 | US 2024/0009091 A1 entire text WO 2022/163812 A1 EP 4286015 A1 CN 116723820 A KR 10-2023-0132790 A | | | |
| JP | 2016-94405 | A | 26 May 2016 | WO 2016/076202 A1 entire text CN 106999385 A | | | |
| JP | 2014-105207 | A | 09 June 2014 | (Family: none) | | | |
| JP | 2020-55806 | A | 09 April 2020 | US 2021/0251875 A1 entire text WO 2020/067559 A1 EP 3797760 A1 KR 10-2021-0005960 A CN 112334125 A | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014136993 A

- JP 2020063239 A

**Non-patent literature cited in the description**

- **A. LEO**. Comprehensive Medicinal Chemistry. Pergamon Press, 1990, vol. 4, 295

- **NISHINO et al.** *Skin Research and Technology*, 2019, vol. 25 (5), 639-652